(19) Europäisches Patentamt
European Patent Office
Office européen des brevets

(11) EP 0 651 636 B1

(12) **EUROPEAN PATENT SPECIFICATION**

(45) Date of publication and mention of the grant of the patent:
**02.10.2002 Bulletin 2002/40**

(21) Application number: **93918397.6**

(22) Date of filing: **26.07.1993**

(51) Int Cl.[7]: **A61K 31/00**, A61K 31/335, A61K 31/20, A61K 31/23, A61K 31/34, A61P 35/00

(86) International application number:
**PCT/US93/07023**

(87) International publication number:
**WO 94/002108 (03.02.1994 Gazette 1994/04)**

(54) **CHEMOTHERAPY FOR CANCER**

VERWENDUNG VON INHIBITOREN DER SYNTHESE VON FETTSÄUREN ZUR BEHANDLUNG VON KREBS

CHIMIOTHERAPIE ANTICANCEREUSE

(84) Designated Contracting States:
**AT BE CH DE DK ES FR GB GR IE IT LI LU MC NL PT SE**

(30) Priority: **24.07.1992 US 917716**

(43) Date of publication of application:
**10.05.1995 Bulletin 1995/19**

(73) Proprietor: **THE JOHNS HOPKINS UNIVERSITY**
**Baltimore, MD 21205-2109 (US)**

(72) Inventors:
- **KUHAJDA, Francis P.**
  **Baltimore, MD 21212 (US)**
- **PASTERNACK, Gary R.**
  **Baltimore, MD 21210 (US)**

(74) Representative: **Dean, John Paul et al**
**Withers & Rogers,**
**Goldings House,**
**2 Hays Lane**
**London SE1 2HW (GB)**

(56) References cited:
**EP-A- 0 246 734** **DD-A- 252 616**
**US-A- 4 328 246**

- **CANCER LETT. vol. 42 , 1988 pages 231 - 235 'Changes in host liver fatty acid synthase in tumor-bearing mice'**
- **JPN.J.EXP.MED. vol. 56, no. 3 , 1986 pages 99 - 106 'Effect of Cerulenin, an Inhibitor of Fatty acid synthesis, on the Immune Cytolysis of Tumor Cells'**
- **PATENT ABSTRACTS OF JAPAN vol. 14, no. 322 (C-739)10 July 1990 & JP,A,02 113 850 (NIPPON OIL & FATS CO. LTD.) 26 April 1990**
- **Derwent Publications Ltd., London, GB; AN 85028975 & JP,A,59 225 115 (OTA SEIYAKU KK) 18 December 1984**
- **PATENT ABSTRACTS OF JAPAN vol. 14, no. 566 (C-789)17 December 1990 & JP,A,02 247 125 (IKEGAWA NOBUO) 2 October 1990**
- **Derwent Publications Ltd., London, GB; AN 85119070 & JP,A,60 058 917 (RIKAGAKU KENKYUSHO) 5 April 1985**
- **PATENT ABSTRACTS OF JAPAN vol. 13, no. 384 (C-629)24 August 1989 & JP,A,01 132 542 (NIWA MASATAKE) 25 May 1989**
- **CANCER CHEMOTHER. PHARMACOL. vol. 14 , 1985 pages 202 - 205 'Inhibition of growth of leukemic cells by inhibitors of calmodulin: phenothiazines and melittin'**
- **ARCH.DE FARMACOL. Y TOXICOL. vol. III, no. 3 , 1977 pages 231 - 240 'Comparative effects of diphenylglyoxal and its superoxide on experimental tumors'**
- **J.BACTERIOL. vol. 98, no. 1 , 1969 pages 23 - 8 'Effects of some antitumor agents on growth and glycolytic enzymes of the Flagellate Crithidia'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **REV ROUM. BIOCHIM. vol. 8, no. 2 , 1971 pages 117 - 22 'Mécanismes bioquimiques impliqués dans la sensibilisation des organismes vivants par des agents chimiques à l'action des radiations et des cytostatiques. II'**
- **ANN. NEW YORK ACAD. SCI. vol. 595, 1990, pages 67 - 73**
- **AM. J. CLIN. PATHOL vol. 97, no. 5, May 1992, pages 686 - 691**
- **BREAST CANCER RES. TREAT. vol. 19, no. 2, 1991, page 213**
- **LAB. INVEST. vol. 66, no. 1, 20 March 1992, page 47A**
- **LAB. INVEST. vol. 66, no. 1, 20 March 1992, page 13A**


Remarks:

The file contains technical information submitted after the application was filed and not included in this specification

## Description

This application is a Continuation-In-Part of U.S. Serial No. 07/917,716, filed July 24, 1992, which is a Continuation-In-Part of U.S. Serial No. 07/735,522, filed July 26, 1991, which is a Continuation-In-Part of U.S. Serial No. 07/622,407, filed December 4, 1990, now abandoned, which is in turn a Continuation of U.S. Serial No. 07/297,722, filed January 17, 1989, now abandoned, which are incorporated herein in their entirety by reference.

The work leading to this invention was supported in part by Grant No. RO1 CA 54404 from the National Institutes of Health. The U.S. Government retains certain rights in this invention.

## BACKGROUND OF THE INVENTION

### Field of the Invention

This invention relates to the field of cancer chemotherapy. In particular, this invention contemplates therapeutic agents which are cytotoxic or cytostatic to tumor cells.

### Background Information

Some carcinoma cells are slow to grow, resulting in tumors that do not pose severe short term risk to a patient having such cells. For instance, many prostate cancers progress so slowly that they are only detected after the patient dies from another cause. Such carcinomas are safely left untreated. Other carcinomas metastasize and grow rapidly, resulting in death of the patient. Any treatment that can slow down the growth of these latter, more virulent carcinomas is desired.

Most current methods of cancer therapy include treatment with chemotherapeutic agents that inhibit cell division or radiation therapy that disrupts DNA in dividing cells. However, these treatments also affect normal cells that happen to be dividing or synthesizing DNA at the time of treatment. Therefore, there is a need for alternative treatments which are more specifically targeted to affect the cells of virulent tumors.

Fatty Acid Metabolism

The fatty acid biosynthetic pathway in man is comprised of four major enzymes: acetyl-CoA carboxylase, the rate limiting enzyme which synthesizes malonyl-CoA; malic enzyme, which produces NADPH; citrate lyase, which synthesizes acetyl-CoA; and fatty acid synthase, which catalyzes NADPH-dependent synthesis of fatty acids from acetyl-CoA and malonyl-CoA. The final products of fatty acid synthase are free fatty acids which require separate enzymatic derivatization with coenzyme-A for incorporation into other products. In man, significant fatty acid synthesis may occur in two sites: the liver, where palmitic acid is the predominant product (Roncari, *Can. J. Biochem*., 52:221-230, 1974); and lactating mammary gland where $C_{10}$-$C_{14}$ fatty acids predominate (Thompson, et al., *Pediatr. Res.,* 19: 139-143, 1985). Except for lactation, and cycling endometrium (Joyeux, et al., *J. Clin. Endocrinol. Metab*., 70: 1319-1324, 1990), the fatty acid biosynthetic pathway is of minor physiologic importance, since exogenous dietary fatty acid intake down-regulates the pathway in the liver and other organs (Weiss, et al., *Biol. Chem. Hoppe-Seyler,* 367:905-912, 1986).

In liver, acetyl-CoA carboxylase, malic enzyme and fatty acid synthase are induced in concert by thyroid hormone and insulin via transcriptional activation and repressed by glucagon (Goodridge, *Fed. Proc.,* 45:2399-2405, 1986) and fatty acid ingestion (Blake, et al., *J. Nutr*., 120:1727-1729, 1990). Tumor necrosis factor alpha (TNF) a cytokine with profound effects on lipogenesis, is either stimulatory or inhibitory depending on the cell type studied. TNF markedly inhibits lipogenesis in adipocytes by reduction in acetyl-CoA carboxylase and fatty acid synthase protein synthesis, but is markedly stimulatory in the liver by increasing the level of citrate, which is the primary allosteric activator of the rate limiting enzyme of fatty acid biosynthesis, acetyl-CoA-carboxylase.

In lactating breast, the other major site of fatty acid biosynthesis in humans, fatty acid synthesis is under control of prolactin, estrogen, and progesterone. During pregnancy, progesterone acts as a mitogen to promote breast development and concomitantly down-regulates prolactin receptors, preventing lipid and milk protein synthesis before delivery. After delivery, the fall in estrogen and progesterone levels allows up-regulation of prolactin receptors and subsequent increase in lipogenic enzymes and milk protein production by breast epithelial cells.

Regulation of fatty acid synthase expression in human breast cancer has been studied primarily as a model for progesterone-stimulated gene expression. In contrast to normal lactating breast where progesterone stimulates epithelial cell growth while retarding lipogenic enzyme synthesis, in progesterone receptor (PR) positive human breast carcinomas such as MCF-7, ZR-75-1, and T-47D, progesterone inhibits growth and induces fatty acid synthase production along with other lipogenic enzymes (Chambon, et al., *J. Steroid Biochem.,* 33:915-922 (1989). Progesterone

presumably acts to up-regulate fatty acid synthase expression via the steroid hormone response element as is found in the rat fatty acid synthase promoter (Amy, et al., *Biochem. J*., 271:675-686, 1989), leading to increased FAS mRNA transcription or, by other mechanisms, to increased message stability (Joyeux, et al., *Mol. Endrocinol*., 4:681-686, 1989). Regarding PR-negative human breast cancer cells, a single study reports that fatty acid synthase accounts for about 25% of cytosolic protein in SKBR3 cells but no data regarding its biologic significance or regulation was available (Thompson, et al., *Biochim. Biophys. Acta*, 662:125-130, 1981).

With regard to cytokines and other lipogenic hormones, only scant data are available concerning human breast cancer. For example, TNF has been known to be markedly growth inhibitory to some breast cancer cultures. While TNF is mildly growth inhibitory to primary rat hepatocyte cultures ($ID_{50}$ = 5000 units/ml), some human breast cancer cells such as MCF-7 are extremely growth inhibited ($ID_{50}$ = 40 units/ml) (Chapekar, et al., *Exp. Cell. Res*., 185: 247-257, 1989). The effect of TNF on FAS expression or lipogenic activity in breast cancer cells, however, remains unknown. One study of fatty acid synthase expression in MCF-7 cells using Northern analysis, found that insulin and insulin growth factor-1 were only slightly stimulatory compared to 5-10 fold increases seen with progesterone, while $T_3$ had no effect (Chalbos, et al., *J. Steroid Biochem. Molec. Biol*., 43:223-228, 1992). Overall, regulation of FAS in receptor positive breast cancer has been only cursorily examined, while receptor negative tumors have not been studied.

No association with poor clinical outcome was found for breast or for any other cancers in those few systems where fatty acid synthase expression was studied. In the only study purporting to associate FAS expression with prognosis, fatty acid synthase expression was studied by *in situ* hybridization in 27 breast cancers, finding an association between increased fatty acid synthase mRNA and a higher degree of morphologic differentiation, but without association with estrogen or progesterone receptor status (Chalbos, et al., *J. Natl. Cancer Inst.,* 82:602-606, 1990). It was deduced from these data that fatty acid synthase expression in breast carcinoma is associated with greater degree of morphologic differentiation and therefore presumably with less aggressive tumors. A second study of 87 cases by Northern blotting of fatty acid synthase mRNA found an association of fatty acid synthase expression and young age (premenopausal patients), but again no association with receptor status (Wysocki, et al., *Anticancer Res.,* 10: 1549-1552, 1990). Neither study provided clinical follow-up of their patients; there were no data comparing FAS expression with either disease-free interval or patient survival. Without clinical outcome, no reliable conclusions can be drawn regarding FAS expression and tumor virulence.

These studies stand in contrast to a series of greater than 200 patients from several centers demonstrating a strong association between poor prognosis and expression of a protein of undetermined function (designated OA-519) through measurement of disease-free survival or overall survival (Kuhajda, *N. Engl. J. Med*., 321:636-641, 1989; Shurbaji, et al., *Am. J. Clin. Pathol*, 96:238-242, 1991; Corrigan, et al., *Am. J. Clin. Pathol*., 96:406, 1991; Cote, et al., *Lab. Invest*., 66:13A, 1992; Ziegler, et al., *Am. J. Clin. Oncol*., 14:101-110, 1991).

Nor has fatty acid metabolism been a target of study in cancer therapeutics. Fujii, et al. (1986, *Japan J. Exp. Med*., 56:99-106), used the fatty acid synthase inhibitor cerulenin in combination with exogenous antitumor antibodies to weaken the cell membrane in an attempt to potentiate complement-mediated cell membrane damage via the membrane attack complex. Cerulenin was known to be toxic to cells at high concentration, and Fujii, et al., taught that the cerulenin concentration should be kept low to maintain the selectivity conferred by the humoral immune component of complement-mediated cell lysis. Spielvogel, et al., U.S. Patent 5,143,907, noted that a series of phosphite-borane compounds exhibited both antineoplastic activity and anti-inflammatory activity while lowering serum cholesterol and serum triglycerides. The phosphite-borane compounds are non-specific inhibitors that affect many cellular functions, and so they are not selectively effective against tumor cells. Spielvogel, *et al.* taught that the hypolipidemic effect on serum cholestrol and triglycerides was mediated through more than one mechanism, and the antineoplastic effect was not shown to be related to the hypolipidemic activity.

Several prior art documents disclose the inhibition of growth of cancer cells using anti-cancer agents. However, none of these documents mentions the cytotoxic effects of using fatty acid synthase (FAS) specific inhibitors to suppress tumour cells (M. J. Tisdale and Y. C Leung (1988) *Cancer Letters* **42:** 231-235; JP1132542 (TOOMEN: KK); W. N. Hait *et al* (1985) *Cancer Chemother Pharmacol* **14:** 202-205; P. G. Partida and F. S. Sánchez (1977) *Arch. de Farmacol. y Toxicol.* **III(3):** 231; Weitzen *et al* (1971) Z. *Physiol. Chem.* **348:** 433-442; C. J. Bacchi *et al* (1959) *Journal of Bacteriology* **1969:** 23-28; M. Fumica *et al* (1971) *Rev. roum. Biochim.* **8(2):** 117-122). However, it has been suggested that cerulenin (a known inhibitor of FAS) is equally cytotoxic to both normal and malignant cells Y. Fujii *et al* ((1986) *Japan J. Exp. Med.* **56(3):** 99-106). In addition, DD252616A1 discloses the use of cerulenin as an antibiotic to treat infections in immunocompromised cancer patients.

D. Chalbos *et al* ((1990) *Ann. New York Acad. Sci. (USA)* **595:** 67-73) discloses that FAS levels may be correlated with both extent of differentiation and progestin levels in tumour cells. However, there is no suggestion that FAS levels may be used as a cancer diagnosis marker.

## SUMMARY OF THE INVENTION

It is an object of this invention to provide a medicament for treating carcinoma patients which will reduce the tumor burden of the patient.

It is another object of the invention to provide a medicament for treating virulent carcinomas.

The present invention provides the use of an inhibitor of fatty acid synthesis in the preparation of a pharmaceutical composition for inhibiting, in a human, growth of tumour cells expressing at least one enzyme of the fatty acid biosynthetic pathway, characterised in that the inhibitor specifically inhibits fatty acid biosynthesis by a cell as demonstrated by inhibition of growth *in vitro* of a tumor cell expressing FAS, said inhibition of growth *in vitro* being reversed by oleate, said inhibitor being specifically cytotoxic to said tumour cells.

The tumor cells may express a protein exhibiting fatty acid synthase activity.

The patient may have a carcinoma selected from the group consisting of breast carcinoma, rectal carcinoma, ovarian carcinoma, colon carcinoma and prostate carcinoma.

The fatty acid synthase expression of the patients normal tissue may be down-regulated during treatment, for example, by reducing the caloric intake of the patient, or by administering a composition containing long chain free fatty acid or acyl glyceride to the patient. Such a composition may supply essential fatty acids to the patient.
The inhibitor of fatty acid synthesis may be an inhibitor of an enzyme selected from the group consisting of fatty acid synthase, acetyl CoA carboxylase, citrate lyase and malic enzyme.

Where the enzyme is fatty acid synthase, the inhibitor may be cerulenin.

Where the enzyme is acetyl CoA carboxylase the inhibitor may be 5-(tetradecyloxy)-2-furoic acid (TOFA).

A further inhibitor of lipid biosynthesis may be administered substantially contemporaneously with the pharmaceutical composition.

The inhibitor of lipid biosynthesis may be Triacisin C.

A further embodiment of the invention provides an assay method to aid in determining virulence of tumor cells in a patient, comprising:

a. obtaining a sample from said patient;
b. determining the amount of a protein exhibiting fatty acid synthase activity in said sample, wherein said step of determining further comprises detecting binding between said protein and an antibody which immunologically binds said protein, wherein said antibody is not immunologically cross-reactive with haptoglobin 1 or 2 or with a polypeptide having the amino acid sequence of leu tyr ser gly asn asp val thr asp ile ser asp asp arg phe pro lys pro pro glu ile ala asn gly tyr val glu lys leu phe arg tyr gln cys, expression by tumor cells of a protein exhibiting fatty acid synthase activity being indicative of virulence.

A further aspect of the invention provides an assay method to aid in determining virulence of tumor cells in a patient, comprising:

a. obtaining a sample from said patient;
b. determining the amount of a protein exhibiting fatty acid synthase activity in said sample, wherein said step of determining comprises detecting hybridisation between a nucleic acid probe and mRNA encoding said protein, expression by tumor cells of a protein exhibiting fatty acid synthase activity being indicative of virulence.

In the assay methods according to the invention, the sample may be a sample of tumor tissue, or a biological fluid from the patient.

The tumor cells may be from a breast carcinoma, prostate carcinoma, or ovarian carcinoma.

The present inventors have discovered the prognostic significance of a protein (designated as OA-519) which is expressed in breast cancers and other carcinomas. Particularly virulent carcinomas tend, among other things, to have cells that express OA-519, and this particular protein has been found to have fatty acid synthase activity which appears to be a required enzyme activity for the growth of carcinomas but not necessarily for normal cells. The inventors have further discovered that inhibitors of fatty acid synthase (FAS inhibitors) are cytotoxic to cells that express OA-519. Based on these discoveries, the inventors have developed the present invention consisting in using inhibitors of fatty acid synthesis to reduce tumor burden in carcinoma patients. The present invention is particularly advantageous because medicament comprising inhibitors such as FAS inhibitors is selective for cells expressing fatty acid synthase. FAS is an inducible enzyme that is not generally expressed by normal cells, and as a result, the tumor cells are preferentially affected by the inhibitors.

## BRIEF DESCRIPTION OF THE FIGURES

Figure 1 shows the correlation between OA-519 Expression and Survival in Breast Carcinoma.
Figure 1A shows the prognostic correlation between expression of OA-519 and progesterone receptor (PR) in breast cancer.
Figure 2A shows the correlation between OA-519 expression and disease free survival in prostate cancer.
Figure 2B shows the correlation between OA-519 Expression and Prognosis in Ovarian Carcinoma.
Figure 2C shows the progressive purification of OA-519 from breast carcinoma by polyacrylamide gel eletrophoresis and corresponding Western blots of proteins at various stages in the purification.
Figure 3A shows the 34-amino acid sequence of a peptide immunologically crossreactive with OA-519. The sequence corresponds to the first 34-amino acids encoded by the hpr gene reported by Maeda, J. Biol. Chem., vol. 260, pp. 6698-6709, 1985.
Figure 3B shows Peptide Sequence Analysis of OA-519.
Figure 4 shows that OA-519 Synthesizes Fatty Acids from Acetyl- and Malonyl-CoA.
Figure 5 shows a Dixon Plot of Cerulenin Inhibition of OA-519 Fatty Acid Synthase Activity.
Figure 6 shows that Cerulenin is Growth Inhibitory to Carcinoma Cells But Not To Normal Cells.
Figure 7 shows that Cerulenin Inhibition Increases as OA-519 Enzyme Activity Increases.
Figure 8 shows the correlation between FAS expression and acyl-glyceride synthesis.
Figure 9 shows detection of OA-$519_{FAS}$ expression in tumor cell lines by *in situ* hybridization of riboprobes having FAS sequence.
Figure 10 shows selective expression of OA-519 detected by immunohistochemical staining.
Figure 11A and B show the relative growth inhibition of cerulenin to different mammary carcinoma cell lines.
Figure 12A and B demonstrate that cerulenin concentration correlates with inhibition of acyl-glyceride synthesis but not with inhibition of cholesterol synthesis.
Figure 13 shows lack of correlation between cell proliferation rate and susceptibility to cerulenin inhibition.
Figure 14 shows growth inhibition of cerulenin to prostatic carcinoma lines.
Figure 15 shows selective growth inhibition by TOFA of selective mammary carcinoma lines with higher levels of endogenous fatty acid biosynthesis.
Figure 16 shows the growth inhibitory effects of Triacsin-C and cerulenin on tumor cells expressing OA-$519_{FAS}$.
Figure 17 shows that exogenously added fatty acids can overcome the inhibition of FAS and rescue cells from the growth inhibitory effect of cerulenin.

## DETAILED DESCRIPTION OF THE EMBODIMENTS

I. Discovery of a New, Tumor-related Enzyme Activity

The present inventors have discovered a protein which is specifically immunoreactive with antibodies specific for an epitope found on the peptide shown in Figure 3A, but not on haptoglobin 1 or haptoglobin 2, and they have discovered that the protein is highly correlated with the most virulent carcinomas. This protein antigen has been designated OA-519 also referred to herein as OA-$519_{FAS}$. Studies on the survival of patients having a wide variety of carcinomas have demonstrated that detection of OA-519 in the tumor or the plasma of the patient correlates with poor prognosis to high statistical significance. (See Table 1.)

Table 1.

| OA-519 Expression and Cancer Prognosis (as Determined by Immunohistochemistry) | | | |
|---|---|---|---|
| Tumor Type | Population | Prognostic Association | Reference |
| Breast | 70 (stages 1&2)<br>135 (all stages)<br>49 (stages 1&2) | Disease recurrence 3.92 relative risk<br>Survival, 4.86 relative risk<br>Early (<2yr) recurrence combined with progesterone receptor (p<0.02) | A<br>Martin, et al*<br>B |

A. Kuhajda, et al, N. Engl. J. Med., 321:636-41, 1989.
B. Cote, et al, Mod. Pathol., 5:13A, 1992.
* See Example 1.

Table 1. (continued)

| OA-519 Expression and Cancer Prognosis (as Determined by Immunohistochemistry) | | | |
|---|---|---|---|
| Tumor Type | Population | Prognostic Association | Reference |
| Prostate | 42 (all stages) | Tumor grade ($p<0.006$), tumor volume ($p<0.004$) | C |
| Colon | 27 (all stages) | Distant metastases ($p<0.02$) | D |
| Ovary | 34 (all stages) | Disease recurrence and survival ($p<0.05$) | Kacinski, et al* |

C. Shurbaji, et al, Am. J. Clin. Pathol., 97:686-691, 1992.
D. Redston, et al, Mod. Pathol., 5:47A, 1992.
* See Example 1.

The inventors have isolated OA-519 and determined that the isolated protein exhibits fatty acid synthase activity. OA-519 purified from a human breast carcinoma cell line has peptide sequence homology with rat fatty acid synthase, and OA-519 also has functional characteristics of a fatty acid synthase. Fatty acid synthesis by OA-519 was demonstrated by incorporation of $^{14}C$ malonyl coenzyme A into fatty acids, subsequent esterification of the fatty acids, and analysis by reversed-phase thin layer chromatography. The specific activity of purified OA-519 was determined spectrophotometrically by following the oxidation of NADPH at 340 nm in the presence of acetyl coenzyme A and malonyl coenzyme A. In one determination, the specific activity of OA-519 was measured as 586 nanomoles NADPH oxidized/min/mg protein, which compares favorably with the value of 404 obtained for FAS from human liver.

Fatty acid synthase is a large protein found in the cytosol of cells from particular tissues, including liver and lactating mammary gland, but FAS is not expressed in most normal (non-malignant) adult tissues. Fatty acid synthase in higher organisms is a multifunctional enzyme which is well known to carry out the following seven enzymatic functions on a single molecule (Wakil, S.J., Biochemistry, 28:4523-4530, 1989):

acetyl transacylase
malonyl transacylase
beta-ketoacyl synthetase (condensing enzyme)
beta-hydroxyacyl dehydrase
enoyl reductase
thioesterase

Breast cancer cells have been found to express fatty acid synthase, while most other tumor cells have not been tested for the presence or absence of this enzyme. Rochefort and co-workers have partially cloned FAS from breast cancer cells and found that FAS expression by breast cancer cell lines was correlated with responsiveness to progesterone (Chalbos, et al, J. Biol, Chem., 262:9923-9926, 1987). Based on this evidence, they concluded that cells expressing FAS were from tumors that were less de-differentiated and therefore less virulent (Chalbos, et al, J. Nat'l. Cancer Inst., 82:602-606, 1990). The present inventors have discovered that, contrary to the teaching of Rochefort, presence of OA-519, a protein which exhibits FAS activity, is highly correlated with the most virulent carcinomas.

Using standard in vitro growth inhibition assays, the inventors have demonstrated that inhibitors of OA-519 inhibit growth of carcinoma cells, but have little effect on normal human fibroblasts. Indeed, fibroblasts, which have very low FAS activity, are resistant to FAS inhibitor concentrations that inhibit growth of more than 80% of breast carcinoma cells having high levels of OA-519 activity. Studies with multiple breast, prostate, lung, colon, and ovarian carcinoma cell lines, and normal fibroblasts confirm the correlation between OA-519 synthase activity and growth inhibition by FAS inhibitors. The relationship between drug sensitivity and OA-519 enzyme activity holds for all tumor cell types tested. Thus, inhibition of the fatty acid synthase enzyme, which is highly expressed in the most virulent carcinomas, can inhibit the growth of cells in these tumors.

## II. Treatment Based on Inhibition of Fatty Acid Synthesis

The present invention provides a medicament for ameliorating tumor burden in carcinoma patients whose tumor contains cells that are dependent on endogenously synthesized fatty acid (fatty acid synthesized within the cells). Such cells usually over-express a protein with FAS activity. Tumor burden may be reduced in such patients by administering to the patient one or more inhibitors that interfere with fatty acid synthesis or utilization. These inhibitors are cytotoxic to tumor cells which express FAS, and administration which results in reduction of fatty acid synthesis and utilization by the tissue and/or reduction of FAS activity in biological fluids of these patients will reduce tumor burden.

A. Selection of the Patient Population

The medicament of this invention may be used to treat patients suffering from cancers which have an elevated level of fatty acid synthase. Characteristic carcinomas amenable to treatment include those of bladder, salivary gland, skin adnexae, bile duct, endocervix, ectocervix, and vagina, esophagus, nasopharynx and oropharynx, or those of germ cell origin, and mesothelioma. In particular, carcinomas or adenocarcinomas of the stomach, endometrium, kidney, liver and lung, as well as melanoma are treatable according to this invention. Breast, colon and rectum, prostate, and ovary, are especially suitable types of adenocarcinomas.

The medicament of this invention may be used to treat tumors having cells that express FAS or depend on endogenous fatty acid (synthesized within the cell). Endogenous fatty acid synthesis by such cells will preferably occur at a rate of incorporation greater than 10 fmoles of acetyl-CoA into acyl glyceride per 200,000 cells per minute. Preferred patients may be identified because they have tumors containing cells which express OA-519 or other enzymes of the fatty acid synthesis pathway, such as acetyl CoA carboxylase (ACC), at levels higher than the level found in the surrounding normal (e.g., non-neoplastic) tissue. Such cells are aggressive tumor cells and result in decreased survival, increased metastasis, increased rates of clinical recurrence and overall worsened prognosis.

Tumor cell sensitivity to fatty acid synthesis inhibitors usually varies continuously with FAS levels. Aggressive tumor cells expressing levels of FAS activity greater than 20 femtomoles malonyl CoA incorporated into fatty acid per 200,000 cells per minute may be expected to be sensitive to fatty acid synthase inhibitors. Since many tumor cells are extremely dependent on endogenous fatty acid synthesis, lower FAS activity levels need not exclude a specific tumor as a candidate for therapy with fatty acid synthase inhibitors.

The presence of FAS in cells of the carcinoma may be detected by any suitable method, including activity assays or stains, immunoassays using anti-FAS antibodies, assays measuring FAS mRNA, and the like. Particularly preferred are assays for the presence of OA-519, a protein which is immunologically cross-reactive with the gene product of the hpr gene (Maeda, J. Biol. Chem., vol. 260, pp. 6698-6709, 1985) but not with haptoglobin 1 or 2. Such assays are taught in International Patent Publication WO 90/08324 or U.S. application serial No. 07/735,522, incorporated herein by reference. The most preferred assays are immunoassays for OA-519, either in tissue or in plasma.

Expression of FAS may be determined directly in tumor tissue samples obtained through procedures such as biopsies, resections or needle aspirates, using assays such as immunohistochemistry, cytosol enzyme immunoassay or radioimmunoassay, *in situ* hybridization of nucleic acid probes with mRNA targets having FAS sequences, or direct measurement of enzyme activity. Expression of fatty acid synthase by the tumor may be indirectly measured in biological fluid samples obtained from patients, such as blood, urine, serum, lymph, saliva, semen, ascites, or especially plasma, using any suitable assays. Preferred assays for FAS in biological fluid include enzyme immunoassay or radioimmunoassay.

Cells which depend on endogenously synthesized fatty acids may also be identified by detection of other enzymes of the fatty acid synthesis pathway at levels higher than those found in non-neoplastic tissue surrounding the tumor. In particular, treatment of cells having unexpectedly high levels of acetyl CoA carboxylase is contemplated by the present invention. The presence of these enzymes may be detected by assay methods analogous to those described for FAS.

Cells that require endogenously synthesized fatty acid are widespread among carcinomas, particularly the most virulent carcinomas. While it is preferred that the presence of FAS be determined prior to treatment, the skilled clinician will recognize that such determination is not always necessary. Treatment of a carcinoma patient with an inhibitor of fatty acid synthesis, particularly a FAS inhibitor, which results in reduction of tumor burden demonstrates the presence of FAS in the tumor. Where a carcinoma patient can be successfully treated by the method of this invention, independent determination of FAS may be unnecessary. Such empirical treatment of carcinomas of the type usually found to express FAS is also within the contemplation of this invention.

Fatty acid synthesis inhibitors are also useful in conjunction with other chemotherapeutic agents. Since no presently prescribed cancer chemotherapeutic agents are specifically active against the fatty acid synthase pathway, FAS inhibitors will complement existing anti-cancer drugs, particularly antimetabolic drugs that target other anabolic or catabolic pathways.

FAS expression and the growth inhibitory effect of inhibitors of the fatty acid synthetic pathway are independent of the cell cycle. Therefore, inhibitors of fatty acid synthesis may be expected to be particularly effective in combination with chemotherapeutic agents that target rapidly cycling cells. Alternatively, fatty acid synthesis inhibitors may be administered to supplement a chemotherapeutic regime based on antineoplastic agents known to be effective against the particular tumor type being treated. In particular, use of fatty acid synthesis inhibitors to prevent the growth of a small proportion of undetected but highly virulent cells in conjunction with a therapeutic program using other agents is within the contemplation of this invention.

On the other hand, it is not contemplated that fatty acid synthesis inhibitors will be useful in combination with agents which produce complement-mediated cell damage via the membrane attack complex, whether initiated by

antibody or by the alternative pathway for complement activation (Bhakdi, et al. (1983), "Membrane Damage by Complement," *Biochim. Biophys. Acta*, 737:343:372). Therefore, this invention is not directed to the use of fatty acid synthesis inhibitors in the presence of exogenously supplied agents which activate the complement-dependent membrane attack complex.

B. Inhibition of the Fatty Acid Synthetic Pathway

Carcinoma cells which are dependent on their own endogenously synthesized fatty acid will express FAS. This is shown both by the fact that FAS inhibitors are growth inhibitory and by the fact that exogenously added fatty acids can protect normal cells but not these carcinoma cells from FAS inhibitors. Therefore, preventing synthesis of fatty acids by the cell may be used to treat carcinoma.

Fatty acids are synthesized by fatty acid synthase (FAS) using the substrates acetyl CoA, malonyl CoA and NADPH. Thus, the fatty acid synthesis pathway is usually considered to involve four enzymes -- FAS and the three enzymes which produce its substrates: acetyl CoA carboxylase (ACC), malic enzyme and citrate lyase. Other enzymes which can feed substrates into the pathway, such as the enzymes which produce NADPH via the hexose monophosphate shunt, may also affect the rate of fatty acid synthesis, and thus be important in cells that depend on endogenously synthesized fatty acid. Inhibition of the expression or the activity of any of these enzymes will affect growth of carcinoma cells that are dependent on endogenously synthesized fatty acid. In accordance with this invention, any suitable method for inhibiting fatty acid synthesis by carcinoma cells may be used to reduce tumor burden in carcinoma patients, including especially inhibitors of the activities of any of the four enzymes of the pathway.

The product of FAS is a free $C_{12}$ - $C_{16}$ fatty acid, usually palmitate. Palmitic acid must be further processed to fulfill the cell's need for various lipid components. As used herein, the term "lipid biosynthesis" refers to any one or a combination of steps that occur in the synthesis of fatty acids or subsequent processing of fatty acids to make cellular components containing fatty acids. The first step in this down-stream processing is activation of the fatty acid by coupling it to coenzyme A, which is catalyzed by an enzyme, acyl CoA synthetase.

Inhibition of key steps in down-stream processing or utilization of fatty acids may be expected to inhibit cell function, whether the cell depends on endogenous fatty acid or utilizes fatty acid supplied from outside the cell, and so inhibitors of these down-stream steps may not be sufficiently selective for tumor cells that depend on endogenous fatty acid. However, it has been discovered that administration of a fatty acid synthesis inhibitor to such cells makes them more sensitive to inhibition by inhibitors of down-stream fatty acid processing and/or utilization. Because of this synergy, administration of a fatty acid synthesis inhibitor in combination with one or more inhibitors of down-stream steps in lipid biosynthesis and/or utilization will selectively affect tumor cells that depend on endogenously synthesized fatty acid. Preferred combinations include an inhibitor of acyl CoA synthetase combined with an inhibitor of FAS or ACC.

C. Inhibitors of Fatty Acid Synthesis

When it has been determined that a patient has a tumor which expresses FAS, or if OA-519 has been found in a biological fluid from a cancer patient, the patient may be treated according to this invention by administering a fatty acid synthesis inhibitor to the patient. Inhibitors whose administration is within the contemplation of this invention may include any compound that shows demonstrable inhibition of lipid biosynthesis or utilization by a cell and which inhibition of cell growth in vitro is reversed by oleate.

Any such compound that inhibits fatty acid synthesis may be used to inhibit tumor cell growth, but of course, compounds administered to a patient must not be equally toxic to both malignant and normal (non-malignant) cells. Preferred inhibitors for use in the method of this invention are those with high therapeutic indices (therapeutic index is the ratio of the concentration which affects normal cells to the concentration which affects tumor cells). Inhibitors with high therapeutic index can be identified by comparing the effect of the inhibitor on two cell lines, one non-malignant line, such as a normal fibroblast line, and one carcinoma line which has been shown to express high levels of OA-519. In particular, therapeutic index may be determined by comparing growth inhibition of animal cells such as human cell lines exhibiting a low level of fatty acid synthesis activity, preferably less than about 10 fmole acetyl-CoA incorporation into acyl glyceride per minute per 200,000 cells, to growth inhibition of human cancer cells exhibiting a high level of fatty acid synthetic activity, preferably greater than about 20 fmole acetyl-CoA incorporation per 200,000 cells per minute, more preferably at least about 80 fmole acetyl-CoA incorporation into acyl glyceride per 200,000 cells per minute. Cells with the preferred level of fatty acid synthesis activity are easily obtained by the skilled worker, and examples of publicly available cell lines are provided in Example 7 below. Preferably, the growth inhibition assays are performed in the presence of exogenous fatty acid added to the cell culture medium, for example, 0.5 mM oleic acid complexed to BSA.

Inhibitors can be characterized by the concentration required to inhibit cell growth by 50% ($IC_{50}$ or $ID_{50}$). FAS inhibitors with high therapeutic index will, for example, be growth inhibitory to the carcinoma cells at a lower concen-

tration (as measured by $IC_{50}$) than the $IC_{50}$ for the non-malignant cells. Inhibitors whose effects on these two cell types show greater differences are more preferred. Preferred inhibitors of fatty acid synthesis will have $IC_{50}$ for cells with high fatty acid synthetic activity that is at least ½ log lower, more preferably at least 1 log lower, than the inhibitor's $IC_{50}$ determined for cells with low activity.

When tumors are treated by administration of a synergistic combination of at least one inhibitor of fatty acid synthesis and at least one inhibitor of either the enzymes which supply substrates to the fatty acid synthesis pathway or the enzymes that catalyze downstream processing and/or utilization of fatty acids, the therapeutic index will be sensitive to the concentrations of the component inhibitors of the combination. Optimization of the concentrations of the individual components by comparison of the effects of particular mixtures on non-malignant and OA-519-expressing cells is a routine matter for the skilled artisan. The dose of individual components needed to achieve the therapeutic effect can then be determined by standard pharmaceutical methods, taking into account the pharmacology of the individual components.

The inhibitor of fatty acid synthesis, or the synergistic combination of inhibitors will be administered at a level (based on dose and duration of therapy) below the level that would kill the animal being treated. Preferably, administration will be at a level that will not irreversibly injure vital organs, or will not lead to a permanent reduction in liver function, kidney function, cardiopulmonary function, gastrointestinal function, genitourinary function, integumentary function, musculoskeletal function, or neurologic function. On the other hand, administration of inhibitors at a level that kills some cells which will subsequently be regenerated (e.g., endometrial cells) is not necessarily excluded.

Acetyl CoA carboxylase and the condensing enzyme of the FAS complex are likely candidates for inhibition. Fatty acid synthesis would be reduced or stopped by inhibitors of these enzymes. The result would be deprivation of membrane lipids, which would cause cell death. Normal cells, however, would survive as they are able to import circulating lipid. Acetyl CoA carboxylase is the focal point for control of lipid biosynthesis. The condensing enzyme of the FAS complex is well characterized in terms of structure and function; the active center contains a critical cysteine thiol, which is the target of antilipidemic reagents, such as cerulenin.

A wide variety of compounds have been shown to inhibit fatty acid synthase (FAS), and selection of a suitable FAS inhibitor for treatment of carcinoma patients is within the skill of the ordinary worker in this art. FAS inhibitors can be identified by testing the ability of a compound to inhibit fatty acid synthase activity using purified enzyme. Fatty acid synthase activity can be measured spectrophotometrically based on the oxidation of NADPH, or radioactively by measuring the incorporation of radiolabeled acetyl- or malonyl-CoA. (Dils, et al, Methods Enzymol, 35:74-83). Suitable FAS inhibitors may be selected, for example, from those exemplified in Table 2, provided that their inhibiting effect can be reversed by oleate.

Table 2.

Representative Inhibitors Of The Enzymes Of The Fatty Acid Synthesis Pathway

Inhibitors of Fatty Acid Synthase:

- 1,3-dibromopropanone
- Ellman's reagent [5,5'-dithiobis(2-nitrobenzoic acid), DTNB]
- 4-(4'-chlorobenzyloxy) benzyl nicotinate (KCD-232)
- 4-(4'-chlorobenzyloxy) benzoic acid (MII)
- 2[5(4-chlorphenyl)pentyl]oxirane-2-carboxylate (POCA) and its CoA derivative
- ethoxyformic anhydride
- thiolactomycin
- cerulenin
- melarsoprol
- iodoacetate
- phenylarsineoxide
- pentostam
- melittin
- methyl malonyl CoA

Inhibitors for citrate lyase:

- (-) hydroxycitrate
- (R,S)-S-(3,4-dicarboxy-3-hydroxy-3-methyl-butyl)-CoA
- S-carboxymethyl-CoA

Inhibitors for acetyl CoA carboxylase:

- sethoxydim

Table 2. (continued)

Representative Inhibitors Of The Enzymes Of The Fatty Acid Synthesis Pathway

Inhibitors for acetyl CoA carboxylase:

- haloxyfop and its CoA ester
- diclofop and its CoA ester
- clethodim
- alloxydim
- trifop
- clofibric acid
- 2,4-D mecoprop
- dalapon
- 2-alkyl glutarate
- 2-tetradecanylglutarate (TDG)
- 2-octylglutaric acid
- 9-decenyl-1-pentenedioic acid
- decanyl-2-pentenedioic acid
- decanyl-1-pentenedioic acid
- (S)-ibuprofenyl-CoA
- (R)-ibuprofenyl-CoA
- fluazifop and its CoA ester
- clofop
- 5-(tetradecycloxy)-2-furoic acid
- beta, beta'-tetramethylhexadecanedioic acid
- tralkoxydim
- free or monothioester of beta, beta prime-methyl-substituted hexadecanedioic acid (MEDICA 16)
- alpha-cyano-4-hydroxycinnamate
- S-(4-bromo-2,3-dioxobutyl)-CoA
- p-hydroxymercuribenzoate (PHMB)
- N6,O2-dibutyryl adenosine cyclic 3',5'-monophosphate
- N6,O2-dibutyry adenosine cyclic 3',5'-monophosphate
- N2,O2-dibutyryl guanosine cyclic 3',5'-monophosphate
- CoA derivative of 5-(tetradecyloxy)-2-furoic acid (TOFA)
- 2,3,7,8-tetrachlorodibenzo-p-dioxin

Inhibitors for malic enzyme:

- periodate-oxidized 3-aminopyridine adenine dinucleotide phosphate
- 5,5'-dithiobis(2-nitrobenzoic acid)
- p-hydroxymercuribenzoate
- N-ethylmaleimide
- oxalyl thiol esters such as S-oxalylglutathione
- gossypol
- phenylglyoxal
- 2,3-butanedione
- bromopyruvate
- pregnenolone

The drug melarsoprol is a trivalent arsenical compound; Pentostam is a pentavalent antimony compound. Trivalent arsenicals react with adjacent thiol groups as do pentavalent antimonials. Fatty acid synthase activity requires multiple reduced thiol groups which would act as targets for inhibition by melarsoprol and other SH reagents.

Aside from these anti-parasite drugs, there are a host of other compounds which inhibit FAS at a variety of sites: protein kinase inhibitors block transcription; colchicine interference with microtubules blocks insulin induction of FAS; melittin, a peptide from bee venom cross-links to the acyl carrier protein of FAS from some species; and cerulenin, an antibiotic, blocks the condensing enzyme activity of FAS. Cerulenin is a specific inhibitor of the condensing enzyme activity of fatty acid synthase as demonstrated by (Funabashi, et al J. Biochem, 105:751-755, 1989) and cerulenin is

a preferred FAS inhibitor for this invention.

Preferred inhibitors of the condensing enzyme include a wide range of chemical compounds, including alkylating agents, oxididents, and reagents capable of undergoing disulphide interchange. The binding pocket of the enzyme prefers long chain, E, E, dienes such as:

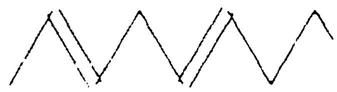

= R

In principal, a reagent containing the sidechain diene shown above and a group which exhibits reactivity with thiolate anions could be a good inhibitor of the condensing enzyme. Cerulenin (2S) (3R) 2,3-epoxy-4-oxo-7,10 dodecadienoyl amide is an example:

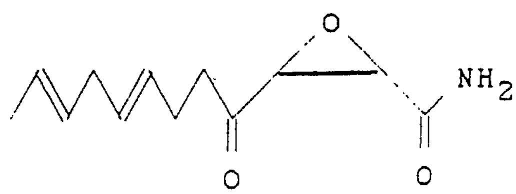

Examples of alternative compounds with different functional groups and the diene sidechain are shown below:

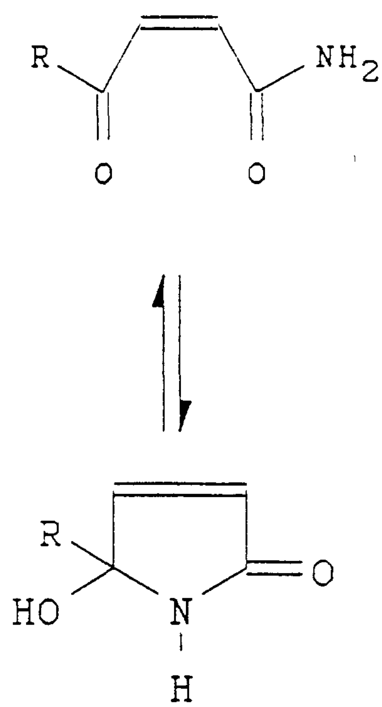

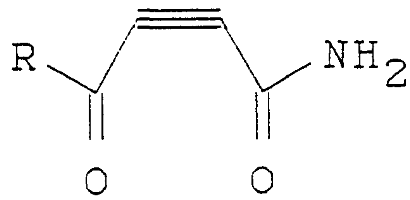

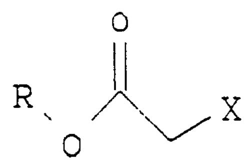

X = Tosyl, halide or other leaving group

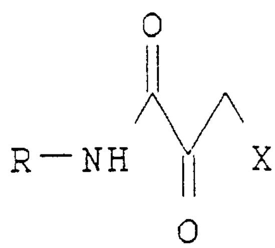

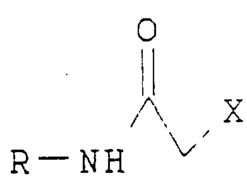

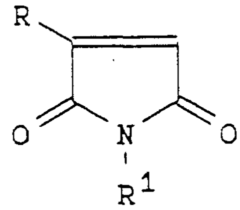

$R^1$= alky

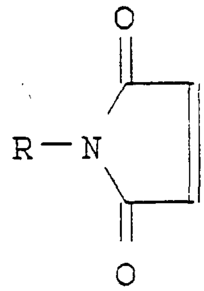

The R group tail can be varied according to the report of Morisaki, et. al. [*Eur. J. Biochem.* 211, 111 (1993)]. Increasing or decreasing the length of the sidechain reduces the inhibitory potency. Tetrahydrocerulenin is 80-150 times less potent than cerulenin. This result is consistent with the idea of $\pi$ electrons in the side chain being of importance in bonding. Also, the trans double bonds confer conformational rigidity which may also be important.

In an alternative embodiment of this invention, compounds are used which inhibit either acetyl CoA carboxylase, malic enzyme or citrate lyase. Representative inhibitors of these enzymes are shown in Table 2. The considerations for selection of the particular inhibitor are the same as discussed above for FAS inhibitors.

Assays for acetyl-CoA carboxylase are taught in U.S. Patent 5,143,907, incorporated herein by reference, and these assays can be used by the skilled worker to determine the inhibitory constants for ACC inhibitors by well-known procedures.

Propanoates which inhibit acetyl CoA carboxylases from diverse organisms are preferred inhibitors. The inhibitors may be represented by the general structure shown below:

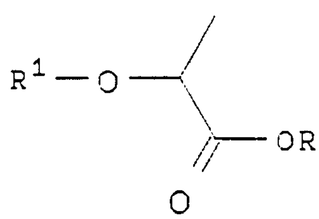

R can be hydrogen, alkyl, or aryl. The configuration at the asymmetric carbon atom can be R, S, or racemic. The acetyl CoA carboxylase in plants is often more susceptible to the R isomer. $R^1$ is often aryl-oxy-aryl:

Ar—O—Ar—

The aromatic rings can be benzene, pyridine, etc. Halo- and other substituents on the aromatic rings are permissible. Examples of propanoates are shown below and/or listed in the Table 2:

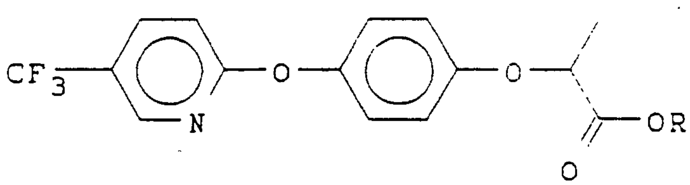

Fluazifop

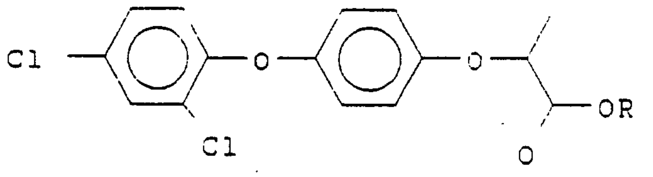

Diclofop

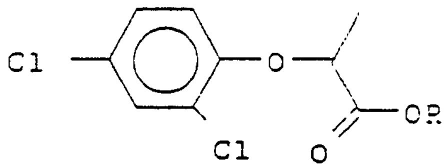

**Dichlorprop**

Some homologs of propanoates are good inhibitors. An example is TOFA, 5 (tetradecyloxy)-2-furoic acid, a potent acetyl CoA carboxylase inhibitor. The structure is shown below:

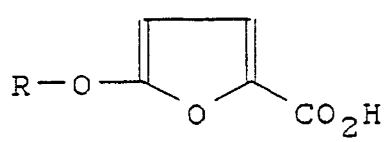

C-2 in this case is not chiral. The R group is a linear saturated 14-carbon sidechain. Methods of synthesizing this compound and related compounds that are also contemplated by this invention are taught in U.S. Patent 4,146,623, incorporated herein by reference.

Another example of a homolog of the propanoates is TDGA or tetradecylglycidic acid:

R = $CH_3(CH_2)$-$_{13}$

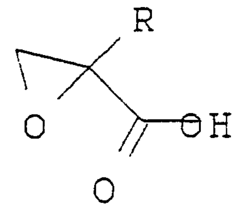

Hydrophobic character and a carboxyl carbon beta to an ether oxygen are common structural traits. Other relevant 2-substituted propanoates include compounds such as ibuprofen, ibuproxam and derivatives thereof.

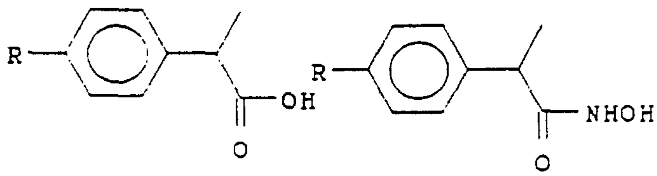

Ibuprofen    Ibuproxam

R = Isobutyl    R = Isobutyl

Ketocylohexenes represent another class of acetyl CoA carboxylase inhibitors. One example is sethoxydim:

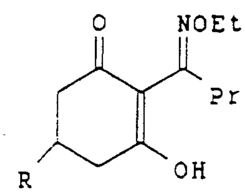

Sethoxydim

Where R is an ethylthiopropyl group.

Another class of compounds which inhibit acetyl CoA carboxylases is represented by the general structure:

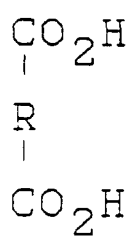

Specific examples such as glutaric acid and pentenedioic acids are listed in Table 2.

In addition to acetyl CoA carboxylase and FAS, other target enzymes include citrate lyase and malic enzyme. These enzymes provide acetate and NADPH for lipid biosynthesis via FAS. The respective reactions are as follows:

Citrate + ATP + CoA → <u>Ac-CoA</u> + ADP + Oxaloacetate

▲

Citrate lyase

Malate + NADP → Pyruvate + $CO_2$ + <u>NADPH</u>

▲

Malic Enzyme

Therapeutic compounds could also be based on these inhibitors as the deprivation of acetyl CoA or NADH would also stop the lipid synthesis.

Of the enzymes in the fatty acid synthetic pathway, FAS is the preferred target for inhibition because it acts only within the pathway to fatty acids, while the other three enzymes are implicated in other cellular functions. Therefore, inhibition of one of the other three enzymes is more likely to affect normal cells. However, where an inhibitor for one of these enzymes can be shown to have a high therapeutic index as described above, the inhibitor may be used therapeutically according to this invention. The skilled clinician will be able to administer inhibitors of any enzyme in the synthetic pathway for fatty acids to treat carcinoma patients in need of such treatment, based on the teaching below.

This invention does not contemplate the use of inhibitors that are generally inhibitory to a large number of different cellular enzyme systems and pathways, such as the phosphite-boranes disclosed in U.S. Patent 5,143,907, or iodoacetamide unless the particular inhibitor can be made relatively specific for lipid biosynthesis as shown by a high therapeutic index (for example, as part of a synergistic combination discussed above).

Palmitate is the major product of the fatty acid synthetase pathway. The elongation and oxidation of palmitate may be critical for production of necessary membrane lipids. For that purpose, the elongation and oxidation steps and any other processing steps for fatty acids would be likely molecular targets for therapeutics. To be incorporated into lipids, both endogenously synthesized fatty acids and exogenous dietary fatty acids must first be activated by acyl-CoA synthetase. Long-chain fatty acyl-CoA is an essential metabolite for animal cells, and so acyl-CoA synthetase is a preferred target.

Tomoda and colleagues (Tomoda *et. al.*, Biochim. Biophys. Act 921:595-598 1987; Omura *et. al.*, J. Antibiotics 39:1211-1218 1986) describe Triacsin C (sometimes termed WS-1228A), a naturally occurring acyl-CoA synthetase inhibitor, which is a product of Streptomyces sp. SK-1894. The chemical structure of Triacsin C is 1-hydroxy-3-(E, E, E-2', 4', 7' - undecatrienylidine) triazene. Triacsin C causes 50% inhibition of rat liver acyl-CoA synthetase at 8.7 μM; a related compound, Triacsin A, inhibits acyl CoA-synthetase by a mechanism which is competitive for long-chain fatty acids. Inhibition of acyl-CoA synthetase is toxic to animal cells. Tomoda *et. al.* (Tomoda *et. al.*, J. Biol. Chem. 266: 4214-4219, 1991) teaches that Triacsin C causes growth inhibition in Raji cells at 1.0 μM, and have also been shown to inhibit growth of Vero and Hela cells. Tomoda *et. al.* further teaches that acyl-CoA synthetase is essential in animal cells and that inhibition of the enzyme has lethal effects.

Lipid synthesis consists of multiple enzymatic steps. The data demonstrate that inhibition of lipid biosynthesis at two or more steps can create synergistic effects, lowering both the required concentration and the potential toxicity of any single agent. Since acyl-CoA synthetase is a ubiquitous enzyme apparently required by all cells for their continued well being, its inhibitors are potentially too toxic to be used effectively as a single anti-cancer agent. In contrast, when acyl-CoA synthetase inhibitors are paired with cerulenin, a specific fatty acid synthase inhibitor, synergistic effects are obtained, rendering each drug more effective.

D. <u>Administration of Inhibitors of Fatty Acid Synthesis</u>

Inhibitors of fatty acid synthesis are preferably formulated in pharmaceutical compositions containing the inhibitor and a pharmaceutically acceptable carrier. The pharmaceutical composition may contain other components so long as the other components do not reduce the effectiveness of the synthesis inhibitor so much that the therapy is negated. Pharmaceutically acceptable carriers are well known, and one skilled in the pharmaceutical art can easily select carriers suitable for particular routes of administration (<u>Remington's Pharmaceutical Sciences</u>, Mack Publishing

Co., Easton, PA, 1985).

The pharmaceutical compositions containing any of the inhibitors of this invention may be administered by parenteral (subcutaneously, intramuscularly, intravenously, intraperitoneally, intrapleurally, intravesicularly or intrathecally, topical, oral, rectal, or nasal route, as necessitated by choice of drug, tumor type, and tumor location.

Dose and duration of therapy will depend on a variety of factors, including the therapeutic index of the drugs, tumor type, patient age, patient weight, and tolerance of toxicity. Dose will generally be chosen to achieve serum concentrations from 0.1 μg/ml to 100 μg/ml. Preferably, initial dose levels will be selected based on their ability to achieve ambient concentrations shown to be effective in in-vitro models, such as that used to determine therapeutic index, and in-vivo models and in clinical trials, up to maximum tolerated levels. Standard procedure in oncology requires that chemotherapy be tailored to the individual patient and the circulatory concentration of the chemotherapeutic agent be monitored regularly. The dose of a particular drug and duration of therapy for a particular patient can be determined by the skilled clinician using standard pharmacological approaches in view of the above factors. The response to treatment may be monitored by analysis of blood or body fluid levels of fatty acid synthase, measurement of FAS activity or OA-519 levels in tumor tissue or monitoring tumor burden in the patient. The skilled clinician will adjust the dose and duration of therapy based on the response to treatment revealed by these measurements.

E. Selective Chemotherapeutic Method

In a preferred embodiment, the medicament of this invention also protects normal cells of patients treated with fatty acid synthesis inhibitors. To protect non-neoplastic normal tissues such as liver (which normally may express wide ranges of fatty acid synthase activity) from potential toxicity, the level of FAS enzyme and/or fatty acid synthetic activity may be down-regulated before and/or during therapy. Down regulation may be accomplished by supplying essential fatty acids in the diet, by reduction of caloric intake or by other effective methods, such as administration of glucagon.

Because FAS is an inducible enzyme in normal tissues, reduction in caloric intake will result in lower expression of FAS by normal cells. The most virulent tumor cells express FAS (OA-519) constitutively. In a patient with limited caloric intake, FAS expression is limited to tumor cells, and the cytotoxic effect of FAS inhibitors will be similarly limited. Down-regulation of FAS expression is usually coupled to fatty acid synthesis inhibitor therapy by reducing caloric intake of the patient before and during administration of the inhibitor.

Another suitable method of reducing FAS expression is exogenous administration of fatty acids, preferably, essential fatty acids. These fatty acids may be formulated in any way that results in the down-regulating FAS expression of normal cells. This could be by including them in the diet of the patient or by formulating them in the same pharmaceutical composition as the fatty acid synthesis inhibitor, or any other suitable method.

Diets suitable for reducing FAS expression in normal tissue are easily within the skill of the ordinary clinician. Any method of reducing FAS expression by normal cells is within the contemplation of the method of this invention, as long as the FAS level in normal cells is reduced during the time that the fatty acid synthesis inhibitor is present in the patient at levels that would be cytotoxic to tumor cells.

## EXAMPLES

The following Examples are provided for purposes of illustration only.

**Example 1.** OA-519 expression is associated with decreased survival in breast carcinoma and decreased survival and disease-free survival in prostate and ovarian carcinoma.

Example 7 of International Patent Publication WO 90/08324 or U.S. Serial No. 07/735,522, incorporated herein by reference, demonstrated that OA-519 expression in breast carcinoma was associated with increased disease recurrence. Clinical studies confirm that OA-519 expression is associated with reduced overall survival in prostate and breast carcinoma and reduced overall and disease-free survival in ovarian carcinoma.

Breast Carcinoma

**Patient Population:** An inception cohort (patients entered into the study at the time of initial surgical treatment) of one hundred and thirty-five women with breast cancer were identified by the Norton Hospital tumor registry, all of whom were treated with mastectomy for primary infiltrating ductal breast carcinoma. The average patient age was 52 and ranged from 32 to 72 years. The average follow-up was 12.3 years and ranged from 10 to 16 years. Patients were admitted to the study when post-surgical treatment records, cause of death, survival time, and paraffin blocks of primary tumor were available for each patient. Estrogen and progesterone receptor information was determined immunohistochemically. In addition, patient age, dose and type of chemotherapy, radiotherapy, and hormonal therapy were documented. Type of infiltrating tumor and nuclear grade were also assessed using the criteria of Fisher et al (Fisher, et al, Cancer, 46:908-918, 1980).

**Immunohistochemical Staining for OA-519:** Immunohistochemical staining used monoclonal anti-OA-519 antibodies from hybridoma cells designated OA-519-M1 or HPR-2, which were deposited with the American Type Culture Collection, 12301 Parklawn Drive, Rockville, Maryland 20852, on July 26, 1991, under ATCC Accession No. HB 10853.

Briefly, the primary anti-OA-519 monoclonal antibody was incubated on the deparaffinized tissue sections at 2.5 ug/ml for 1 hour at 37°C. Following rinsing in rinse buffer, the slides were then incubated in 1/400 rabbit anti-mouse antibody (DAKO) for 1 hour. Following another rinse, the slides were incubated with avidin-linked horseradish peroxidase (Vectastaing ABC kit) for 1 hour. After the avidin-biotin complex formation, the slides were incubated in aqueous hematoxylin, coverslipped and observed.

**OA-519 Immunoreactivity and Criteria for Positivity:** Positive staining was finely granular, cytoplasmic, and heterogeneous. Additionally, staining either had to be visible at 100 X magnification, or label at least 10% of tumor cells for a case to be scored positive.

**Prognostic significance of OA-519 Immunoreactivity:** Patients whose tumor-stained positively for OA-519 had a markedly increased risk of dying of breast carcinoma. Figure 1 is a life table which shows the fate of OA-519 positive and negative patients. For example, after 12 years, about 37% of the OA-519 negative patients were dead compared to approximately 85% of the OA-519 positive patients.

The following table shows the significance of OA-519 reactivity by stage:

| | STAGE 1 | STAGE 2 | STAGE 3 |
|---|---|---|---|
| | % dead | % dead | % dead |
| OA-519+ | 9/13 (70%) | 27/31 (87%) | 9/9 (100%) |
| OA-519- | 3/20 (15%) | 16/41 (39%) | 12/21 (57%) |
| p-value | 0.002 | < 0.0001 | 0.019 |

**Expression of OA-519 by Prostatic Adenocarcinoma**

OA-519 expression in prostate cancer was also found to be associated with disease recurrence. Patients having been diagnosed and treated for prostate adenocarcinoma were selected from the files of the Mountain Home VA Medical Center. The study population included 99 patients with prostate cancer in American Urologic System (AUS) stages A through D1. Clinical Stage information was obtained from the tumor registry abstracts or by review of the clinical records. Patients were excluded from the study is they had distant metastasis at the time of presentation (AUS stage D2), their status at last follow up was unknown, or if the total follow up was less than two years.

Histopathologic Studies:

Tumor grading: All slides were reviewed and a Gleason score was determined by adding the numbers for the two most predominant patterns (Gleason, in Tannenbaum M (ed): Urologic pathology: The prostate. Philadelphia, 1988, Lea & Febiger, pp. 171-198.). Gleason scores 2-4 were assigned Grade I, scores 5-7 were assigned grade II, and scores 8--10 were assigned Grade III.

Immunohistochemical Studies

A single representative tissue block was selected from each cancer for immunohistochemical staining. An affinity purified polyclonal antibody raised in rabbits against purified OA-519 was used in this study. Staining was performed on routinely processed, formalin fixed, paraffin embedded tissue. The Avidin-Biotin Complex (ABC) immunoperoxidase technique utilizing unlabeled primary antibody was used. In brief, 6mm deparaffinized and rehydrated tissue sections were incubated in 5% nonfat dry milk in phosphate buffered saline (PBS) including 3% hydrogen peroxide for 20 minutes to block endogenous peroxidase activity as well as non-specific protein interactions. Slides were then incubated with affinity-purified polyclonal anti-OA-519 at 2.7 ug/ml in PBS at pH 7.2 for one hour at room temperature. With intervening washes with PBS, the sections were successively incubated with biotinylated goat anti-rabbit immunoglobulin diluted 1:200 in PBS (Vector Laboratories) and avidin-horseradish peroxidase complex (Vectastain$^R$, Vector Laboratories), both for 30 minutes at 22° C. Aminoethylcarbazole (AEC) (Vector Laboratories) was used as the chromogen with Mayer's hematoxylin counterstain. For negative controls, PBS was substituted for primary antibody for

each case. A known anti-OA-519 positive case was used as a positive control with every run.

Staining was defined as positive for OA-519 epitopes if (1) immunoreactivity was discernible at lower power (100x) (2) granular cytoplasmic staining was present without observable nuclear staining, and (3) staining was heterogeneous (i.e., the level of reactivity varied from cell to cell or from region to region. Tumors were scored as positive or negative. Positive staining for OA-519 was seen in 56 (57%) of the 99 primary prostate cancers examined.

The mean total follow up time was 4.17 years (range, 2.01 - 9.33). Prostate cancer recurred or progressed in 19 (19%) of the patients. Progression was defined as the appearance of local or metastatic disease after "curative" treatment, such as radical prostatectomy or radiation, or advance in the stage of disease in patients treated with hormonal therapy or expectantly managed. The average time to progression of disease was 2.54 years (range 0.67 - 5.85).

There were four (9%) cases of disease progression among the OA-519-negative group compared to 15 (27%) in the OA-519-positive group (Kaplan-Meier plot shown in Figure 24-1). The difference between these groups in the proportion of cancers that progressed was statistically significant (Wilcoxon and log rank tests ($P < 0.009$) and Fisher exact test ($P < 0.04$)). OA-519 was a particularly valuable prognostic indicator among the low and intermediate grade prostate cancers, where the histologic grade by Gleason score was not a significant prognostic indicator (data not shown).

**Ovarian Carcinoma**

An ovarian carcinoma study by Kacinski et al. is in progress using the same antibody, staining procedure, and interpretation that was used in the above breast carcinoma study. However, based on analysis of 34 patients completed so far, there is an association of OA-519 expression with reduced disease-free and overall survival, which is demonstrated in Figure 2B.

**Example 1A: Prognostic Markers**

OA-519 expression was strongly prognostic in early breast cancer, as shown in Example 1. This prognostic potential was independent of the prognostic power of estrogen and progesterone receptors. Increased expression of OA-519 in human breast carcinoma conferred a significantly worsened prognosis as measured either by disease recurrence or overall survival. In a clinical study of 135 patients with Stage I-III breast carcinoma (Martin, et al., manuscript in preparation), Cox multi-variate proportional hazard analysis demonstrated that OA-519 and progesterone receptor expression were most strongly and independently associated with adverse survival regardless of stage (univariate relative risk 4.860, 0.070; multi-variate relative risk 2.567, 0.153, respectively).

The prognostic power of OA-519 expression is further illustrated by the accompanying Kaplan-Meier plots (Figures 1 and 1A). Figure 1 demonstrates that about 10% of OA-519 positive patients survived for 15 years as compared to about 50% of OA-519 negative patients. Figure 1A graphically demonstrates the improved prognostic stratification when the two independent prognostic markers, OA-519 and progesterone receptor, are combined. This allowed stratification of patients into an OA-519 positive, progesterone receptor negative high risk group (88% dead), an OA-519 negative/progesterone receptor positive low risk group (5.4% dead), and an intermediate risk group (63% dead).

Among other markers included in this study, OA-519 was independent of $p185^{neu}$ and cathepsin D expression. Interestingly, OA-519 expression was not associated with tumor cell proliferation as measured by proliferating cell nuclear antigen. In a separate study of OA-519 expression and S-phase determined by flow cytometry, OA-519 expression also showed no association with the S-phase fraction (Shurbaji, et al., *Lab Invest.,* 68:69A, 1993). Therefore, OA-519 expression could be utilized with the aforementioned, or any other independent prognostic marker to improve stratification of the patient population.

**Example 2. Purification and Partial Sequence of OA-519 Protein from Cell Lysates**

OA-519 purified from a human breast carcinoma cell line has peptide sequence homology with rat fatty acid synthase, based on internal peptide sequence from OA-519, obtained by electroblotting a limited proteolytic digest of OA-519 with subsequent microsequencing directly from the PVDF membrane.

OA-519 was purified from the ZR-75-1 human breast carcinoma cell line, as demonstrated in Figure 2C. The Figure shows 4-8% gradient gel SDS PAGE of a protein preparation in various stages of purification. The right hand panel is stained with Coomassie Blue, and the left hand panel is a Western blot using affinity purified polyclonal antibody raised against the peptide shown in Figure 3A.

ZR-75-1 human breast carcinoma cells were grown to near confluence in medium. Confluent monolayers were rinsed with phosphate-buffered saline, then scraped free, harvested by centrifugation, and stored frozen.

To each aliquot of approximately 1.5 x $10^7$ cells, 10 ml of purification buffer (20 mM Tris HCl, pH 7.5 at 4°, 1 mM EDTA, 0.1 mM diisopropylfluorophosphate, 0.1 mM phenylmethylsulfonyl fluoride) was added. Cells were homogenized with 10 strokes of a Dounce homogenizer, then clarified supernate was obtained by centrifugation at 16,000 x g for 30 min. at 4°C. (Lane L of Figure 2C was loaded with clarified ZR-75-1 hypotonic lysate.)

A Sephacryl S-200 (Pharmacia) gel filtration column, 2.5 x 90 cm, was pre-equilibrated with purification buffer, pH=8.0, containing 1 mM β-mercaptoethanol and 100mM KCl. ZR-75 lysate was filtered through 0.45 mM filter, then loaded onto column at 25 ml/h. Fractions were analyzed for presence of 270,000 Da polypeptide by SDS-PAGE using a 4% Coomassie-stained gel. Presence of the polypeptide may optionally be confirmed by using Western blotting with either polyclonal antibody specific for peptide shown in Figure 3A or anti-OA-519 protein, developing the blots with $^{125}$I-protein A. (Lane G of Figure 2C was loaded with pooled fractions from the gel filtration column.)

Positive fractions from the Sephacryl column were pooled, diluted with an equal volume of purification buffer plus 1 mM β-mercaptoethanol then loaded onto a pre-equilibrated Mono-Q HR 5/5 anion exchange HPLC column (Pharmacia). At a flow rate of 1 ml/min., the column was washed with 15 ml of purification buffer plus 1mM β-mercaptoethanol, eluted with linear 60 ml gradient over 60 min. to a final 1 M KCl concentration, then washed with 5 ml of 1 M KCl-containing purification buffer plus 1 mM β-mercaptoethanol. Fractions containing the polypeptide were selected by SDS-PAGE using Coomassie-stained 4% gels. (Lane A in Figure 2C was loaded with pooled fractions from the anion exchange column.) Fractions containing purified polypeptide, designated OA-519, were pooled and further processed according to downstream experimental needs. Characteristic yields were roughly 1 mg of OA-519 per 2 x $10^7$ cells with purity of 98% or greater, estimated by Coomassie stained SDS polyacrylamide gels.

A final hydroxyapatite chromatography step was added to achieve more than 99% purity using a Bio-Rad MAPS Analytical HPHT Cartridge. Using a 0-600 millimolar phosphate gradient, OA-519 elutes in one peak at 200 millimolar phosphate.

Purified OA-519 was dialyzed into 50 millimolar ammonium bicarbonate, pH 8.0 and proteolytically cleaved with a 1:50 dilution of endoproteinase glutamate C (V8 protease) for 15 minutes at 37°C. The peptides were subjected to SDS-PAGE on 4% Laemmli gels and transferred to PVDF membranes (BioRad), and were sequenced directly from the PVDF membrane using automated Edman degradation on an Applied BioSystems gas phase sequencer (Matsudaira, P.T., "A Practical Guide To Protein and Peptide Purification for Micro-Sequencing", Academic Press, New York, 1989).

Limited proteolytic cleavage generated two major peptides of approximately 150 and 134 kD. The 150 kD peptide had a blocked N-terminus and thus represented the N-terminal OA-519 peptide. N-terminal sequence was obtained from the 134 kD internal peptide which demonstrated 84.6% homology with rat fatty acid synthase over 13 amino acids as seen in Figure 3B. Thus, OA-519 has structural homology with fatty acid synthase, also an approximately 270 kD molecule.

Example 3. OA-519 has Fatty Acid Synthase Activity

Purified OA-519 from the ZR-75-1 human breast carcinoma cell line has fatty acid synthase activity based on its ability to incorporate acetyl coenzyme A and malonyl coenzyme A into fatty acids in the presence of NADPH, a reaction specific for fatty acid synthase (Wakil, S.J., Biochemistry, 28:4523-4530, 1989). This reaction is specific for fatty acid synthase. Fatty acid synthesis by OA-519 was demonstrated by incorporation of $^{14}$C malonyl coenzyme A into fatty acids, subsequent esterification of the fatty acids, and analysis by reversed-phase thin layer chromatography.

**Incorporation of $^{14}$C malonyl coenzyme A into fatty acids by OA-519:** OA-519 was purified as in Example 2 except that protease inhibitors were omitted as they interfere with the final step of the synthase assay. 4.2 μg of OA-519 in 20 ul of 20 millimolar Tris HCl, 270 millimolar KCl, 1 millimolar EDTA, 1 millimolar DTT, pH 7.5 at 25°C was added to the following reaction mixture: 75 nanomoles NADPH; 25 nanomoles acetyl coenzyme A; 16.6 μl of 1 molar potassium phosphate, pH 6.6 at 25°C; and 97 ul HPLC grade water to a total volume of 150 μl. The reaction mixture was vortexed and 5 ul of $^{14}$C malonyl coenzyme A (20 uCi/ml; 51 mCi/millimolar) and 25 nanomoles malonyl coenzyme A were added. Following vortexing, the reaction mixture was incubated at 37°C for 20 minutes and stopped by the addition of 1 ml of 1:1 chloroform:methanol.

**Methyl esterification of $^{14}$C fatty acids:** Prior to thin layer chromatographic separation of the $^{14}$C fatty acid mixture, methyl esters of the $^{14}$C fatty acids were prepared using the method of methanolic sulphuric acid. The chloroform:methanol:reaction mixture was vortexed then agitated for 30 minutes. Following centrifugation, the supernatant was dried under $N_2$. The dried lipids were extracted twice in 400 μl of hydrated n-butanol:HPLC water (1:1) pooled, washed, and dried under $N_2$. To synthesize the methyl esters, 0.75 ml of 2% sulfuric acid in methanol was added to the dried fatty acids, gassed with $N_2$, and incubated for 2 h at 70°C. Following the addition of 0.75 ml of HPLC grade water, $^{14}$C fatty acid methyl esters were extracted twice with 1.5 ml of pentane, washed with 0.5 ml HPLC water and dried.

$^{14}$C fatty acid methyl esters were separated and identified using reversed phase thin layer chromatography as follows. Reversed-phase thin layer chromatographic plates (20 x 20 cm, Analtech) were baked in a vacuum oven at 80°C for 20 minutes. Dried $^{14}$C fatty acid methyl esters and standards were resuspended in 20 μl chloroform:meth-

anol (9:1), spotted, and chromatographed in chloroform: methanol:water (5:15:3). Non-radioactive standards were visualized with cyclodextrin spray in iodine vapor. $^{14}C$ fatty acid methyl esters were detected using a Bioscan System 2000 imaging scanner with Autochanger 3000.

Results: OA-519 synthesized 85% palmitate (16 carbon saturated fatty acid), with approximately 6% myristate and 8% stearate (14 and 18 carbon saturated fatty acids, respectively) (Figure 4). These data demonstrate that OA-519 has fatty acid synthase activity by showing generation of complete fatty acids from $^{14}C$-labeled malonyl-CoA. The ratios among product fatty acids is similar to fatty acid synthase from human liver, but markedly different than that for fatty acid synthase from lactating human breast (34% stearate, 33% palmitate, 16% myristate).

Kinetic Characterization of OA-519 Fatty Acid Synthase

The specific activity of purified OA-519 was determined spectrophotometrically by following the oxidation of NADPH at 340 nm in the presence of acetyl coenzyme A and malonyl coenzyme A. OA-519 has a specific activity of 586 nanomoles NADPH oxidized/min/mg protein which compares favorably with the value of 404 obtained for human liver.

Spectrophotometric studies with OA-$519_{FAS}$ demonstrated that the apparent $K_m$ of 86.2 X $10^{-5}$ M for malonyl-CoA was higher than the literature values reported for rat or rabbit mammary gland (1.3 X $10^{-5}$ M or 2.9 X $10^{-5}$ M, respectively) (Smith, et al., *Methods Enzymol.*, 35:65-74, 1975; Dils, et al., *Methods Enzymol.*, 35:74-83, 1975) or for the synthase from the human breast cancer cell line SKBR3 (1.8 X $10^{-5}$ M) (Thompson, et al., *Biochim. Biophys. Acta,* 662:125-130, 1981). The $K_m$ for the purified synthase from normal human tissues has not been reported. In contrast to the $K_m$ values, the specific activity of 624 nmol NADPH oxidized/min/mg protein was similar to the reported specific activities of fatty acid synthases purified from a variety of sources including human liver (Roncari, *Methods Enzymol.*, 71:73-39, 1981).

Example 4: Fatty acid synthesis by OA-519 is inhibited by FAS inhibitors.

Cerulenin is a specific inhibitor of the condensing enzyme activity of fatty acid synthase as demonstrated by Funabashi, et al, J. Biochem., 105:751-755, 1989. The drug melarsoprol is a trivalent arsenical compound; trivalent arsenicals react with adjacent thiol groups. Fatty acid synthase activity requires multiple reduced thiol groups which would act as targets for inhibition by melarsoprol.

Using purified OA-519, in the spectrophotometric enzyme assay, cerulenin was shown to be a non-competitive inhibitor of OA-519 fatty acid synthase activity.

**Spectrophotometric fatty acid synthase assay:** The fatty acid synthase assay was performed in a total volume of 451 µl with the following components: 18.88 µg of purified OA-519 fatty acid synthase from the ZR-75-1 breast carcinoma cell line as described in Example 2, 25 nanomoles acetyl-CoA, 75 nanomoles NADPH, and 50 µl of 1 molar potassium phosphate, pH 6.6 at 25°C. The substrate malonyl-CoA was added at concentrations of 0.5 and 0.6 micromolar in the presence of 0, 9.9, 148, 198, or 397 micromolar cerulenin. HPLC grade water was added to achieve a final volume of 451 µl for all assays.

The enzyme, NADPH, acetyl-CoA, phosphate, cerulenin, and water were combined, vortexed, and incubated in a Beckman DU 650 spectrophotometer at 37°C. Oxidation of NADPH was monitored at 340 nm without substrate to determine background for 2 min. Malonyl-CoA was then added to the reaction cuvette, mixed, and incubated at 37°C while NADPH oxidation was determined at 10 second intervals at 340 nm in the spectrophotometer for a total of 2 min. All determinations were performed in duplicate.

**Results:** The spectrophotometric data was plotted according to Dixon (Dixon, M., Biochem. J., 55:170-171, 1953) which graphs inhibitor concentration versus 1/v for each given substrate concentration. The analysis in Figure 5 demonstrates that cerulenin inhibits the OA-519 fatty acid synthase with an apparent $K_i$ of 32 micromolar for the substrate malonyl-CoA. The inhibition also appears to be non-competitive as has been reported for cerulenin (Funabashi, et al, J. Biochem., 105:751-755, 1989), since the curves intersect at the abscissa.

Example 5: Selective Effects of FAS Inhibitors

FAS inhibitors are growth inhibitory to carcinoma cells but not to normal cells. OA-519 is overexpressed in breast carcinomas with poor prognosis but little OA-519 expression is identified in normal tissues. Using standard in vitro growth inhibition assays, two inhibitors of OA-519, cerulenin and melarsoprol, were shown to be anti-proliferative to carcinoma cells but to have little effect on normal human fibroblasts.

In vitro growth inhibition assays for OA-519 inhibition were performed using ZR-75-1 cells or normal human fibroblasts which were grown to confluence, and then 25,000 cells were plated in 1:1 conditioned:fresh medium (RPMI with 10% fetal calf serum) supplemented at a physiologic level to 0.5 millimolar oleic acid bound to previously delipidized

bovine serum albumin. Cells were allowed to attach and grow overnight. At time 0, drugs diluted in RPMI medium with 10% fetal calf serum were added to achieve the following concentrations: 50 μg/ml to 0.01 μg/ml with serial two-fold dilutions including no drug and empty well controls. Each concentration was performed in quadruplicate and time points were taken at 4, 8, 24, and 48 h. After drug treatment, cells were stained with crystal violet, solubilized in SDS, and read on a Molecular Diagnostics automated plate reader at 570 nm.

Figure 6 demonstrates that at a cerulenin concentration of 6.25 μg/ml, normal human fibroblasts are unaffected by the drug, whereas by 48 h, growth of the ZR-75-1 cells was over 80% inhibited. Presence of 0.5 millimolar oleic acid in the culture medium prevented cerulenin growth inhibition in the normal fibroblasts, but not in the breast carcinoma cells. In similar experiments without oleic acid supplementation, growth of both breast carcinoma and normal fibroblasts was inhibited.

<u>Example 6.</u> Cytotoxicity of OA-519 inhibition is related to the level of OA-519 synthase activity.

Fibroblasts with vanishingly little OA-519 synthase activity are resistant to cerulenin concentrations which inhibit growth by more than 80% of breast carcinoma cells having high levels of OA-519 activity. To show that carcinoma cell lines which express OA-519 are sensitive to OA-519 inhibitors, while those with low OA-519 activity are resistant, multiple breast, prostate, lung, colon, and ovarian carcinoma cell lines, and normal fibroblasts were studied, correlating OA-519 synthase activity and growth inhibition by cerulenin. The relationship between drug sensitivity and OA-519 enzyme activity held for all cell types.

**OA-519 fatty acid synthase activity:** For each cell type, 200,000 cells were plated in 24 well plates, grown overnight in triplicate. Cells were then scraped, pelleted, and frozen at
-80°C until assayed for OA-519 enzyme activity.

A modified assay was used to measure the OA-519 fatty acid synthase activity, in order to increase the sensitivity by which incorporation of $^{14}C$-malonyl coenzyme A into fatty acid could be measured compared to the spectrophotometric assay used in Example 3. Following a hypotonic lysis of the frozen cell pellets in 1 millimolar DTT, 1 millimolar EDTA, 20 millimolar Tris HCl, pH 7.5 at 25°C, and centrifugation at 14,000 x g for 10 minutes, 20 ul of the lysate is added to a reaction mixture containing 75 nanomoles NADPH; 25 nanomoles acetyl coenzyme A; 16.6 μl of 1 molar potassium phosphate, pH 6.6 at 25°C, 97 μl HPLC grade water to a total volume of 150 μl. Following vortexing, 2 μl of $^{14}C$-malonyl coenzyme A (20 uCi/ml; 51 mCi/millimolar) and 25 nanomoles malonyl coenzyme A were added and vortexed. The reaction mixture was incubated at 37°C for 20 minutes and stopped by the addition of 1 ml of 1:1 chloroform:methanol.

The chloroform:methanol:reaction mixture was vortexed and agitated for 30 minutes. Following centrifugation, the supernatant was dried under $N_2$. The dried lipids were extracted twice in 400 μl of hydrated n-butanol:HPLC water (1:1) pooled, washed, dried under $N_2$, and counted for $^{14}C$.

**Growth Inhibition Assay:** Anti-proliferative activity of cerulenin for each cell line was determined using the same assay and drug concentrations described in Example 5, except that no oleic acid was added and a single 24 h time point was taken.

Figure 7 shows the relationship between OA-519 enzyme activity and sensitivity of the carcinoma cells to the inhibitor cerulenin. At enzyme activity below 4 picomoles malonyl CoA incorporated into fatty acid per 200,000 cells per minute, less than 50% of the cells were growth inhibited (with the exception of the H125 lung carcinoma line, 11.6 picomoles per minute and 48% growth inhibition), In contrast, most cell lines with more than 7 picomoles malonyl CoA incorporated into fatty acid per 200,000 cells per minute showed more than 50% growth inhibition. Thus, increasing OA-519 enzyme activity is associated with increased sensitivity to cerulenin anti-proliferative activity. Cells with increased OA-519 enzyme activity appear to be reliant on the OA-519 fatty acid synthase pathway.

<u>Example 7: OA-519 Fatty Acid Synthase Activity Parallels Acyl-glyceride Synthesis in Cancer Cell Lines and Human Fibroblasts</u>

Table 3 lists a group of 10 human cell lines with high, intermediate, and low FAS enzyme activity which, along with human embryonic fibroblast lines, have been used in the experiments described herein.

<u>OA-519 synthase activity</u>: For each cell type, 200,000 cells were plated in triplicate in standard 24 well plates, grown overnight, scraped, pelleted, and frozen at -80° C. Following hypotonic lysis of frozen pellets in 1 mM DTT, 1 mM EDTA, 20 mM Tris HCl, pH 7.5 at 25° C, 20 ul of the lysate were added to a reaction mixture followed by addition of $^{14}C$ malonyl-CoA. The reaction mixture was incubated at 37° C for 20 min. and stopped by the addition of 1 ml of 1:1 chloroform:methanol. After a 30 min. extraction, the lipids were dried under $N_2$, twice extracted in 400 μl hydrated n-butanol:water, 1:1, pooled, washed, dried under $N_2$, and counted for $^{14}C$. Activity was normalized to cell number or to total cellular protein. The results are shown in Table 3.

Table 3

| Characteristics Of Cell Lines Utilized In In-Vitro Experiments | | | | |
|---|---|---|---|---|
| CELL LINE | ORIGIN | CULTURE MEDIUM | FAS ACTIVITY | |
| SKBR3 | Human Breast ER- | McCoy's + 15% FCS | High[a] | 198.04 |
| ZR-75-1 | Human Breast, ER+ | RPMI + 10% FCS | Intermediate[a] | 38.08 |
| MCF-7 | Human Breast, ER+ | DMEM + 10% FCS | Intermediate[a] | 31.27 |
| MCF-7a | Human Breast, adriamycin Resistant | DMEM + 10% FCS | Low[a] | 18.32 |
| HS-27 | Human Fibroblast | DMEM + 10% FCS | Low[a] | 13.87 |
| SW-480 | Human Colon | Laibovitz + 10% FCS | Low[a] | 7.93 |
| LNCAP | Human Prostate Androgen responsive negative | DMEM + 10% FCS | High[b] | 700 |
| TSU-pr1 | Human Prostate Androgen responsive negative | DMEM + 10% FCS | High[b] | 700 |
| Dupro 1 | Human Prostate Androgen responsive negative | DMEM + 10% FCS | Intermediate[b] | 400 |
| DU-145 | Human Prostate Androgen responsive negative | DMEM + 10% FCS | Intermediate[b] | 400 |
| PC3 | Human Prostate Androgen responsive negative | DMEM + 10% FCS | Low[b] | 140 |

a. fmol of 14-C-malonyl-CoA incorporated into fatty acids per 200,000 cells per minute.

b. fmol of 14-C-malonyl-CoA incorporated into fatty acids per μg protein per minute.

Further assays were done to determine whether increased FAS levels were correlated with increased overall fatty acid biosynthesis. Because acetyl CoA carboxylase, the rate-limiting enzyme in the pathway, lies proximal to fatty acid synthase, analysis of acyl-glyceride production from $^{14}$C-acetate may be measured as a reliable indicator of overall pathway activity.

Measurement of endogenous fatty acid incorporation into acyl-glycerides: For each cell type, 200,000 cells were plated in triplicate for non-polar and polar lipid analysis. Following overnight growth, each well of cells was incubated for 2 hours with 1 μCi $^{14}$C acetate. $^{14}$C-labeled lipids were extracted as above, and dried under $N_2$. For analysis of non-polar lipids, samples were resuspended in 10 ul chloroform and spotted on silica gel N-HR (Brinkmann) and chromatographed in hexane:ethyl ether:acetic acid, 65:25:4 by volume. Cholesterol, palmitic acid, tripalmitin, and cholesterol palmitate (Matreya) were run as controls at 50 μg each. For analysis of polar lipids, samples were spotted on silica get 6A (Whatman) and chromatographed in hexane:ethyl ether:acetic acid 90:10:1 by volume. 50 μg each of cholesterol, phosphatidyl ethanolamine, lecithin, and lysolecithin (Matreya) were run as controls. $^{14}$C-labeled lipids were detected and quantified using a Bioscan System 2000 imaging scanner with Autochanger 3000. Non-radioactive standards were visualized using rhodamine spray under ultraviolet light. Error bars on Figure 8 represent the standard error of the mean. Phosphatidylcholine and triglycerides were the predominant acyl-glycerides detected.

As shown in Figure 8, FAS activity (as measured by $^{14}$C-malonyl-CoA incorporation into total lipids) correlated strongly ($r^2$=0.93) with the overall fatty acid byosynthetic pathway activity (as measured by the rate of $^{14}$C-acetate incorporation into acyl-glycerides). Importantly, TLC analysis demonstrated that during the time course of the experiment, most of the $^{14}$C-acetate was incorporated into phosphatydylcholine with variable quantities of triglycerides or phosphatidylethanolamine. No $^{14}$C-labelled cholesterol esters, free fatty acid, or phosphatidylserine were detected. These data mean that (a) increased FAS activity indicates increased overall fatty acid synthesis, (b) phosphatidylcholine, a major membrane lipid, was the predominant end product of FAS, and (c) the activities of acetyl CoA carboxylase and malic enzyme, which are co-regulated with FAS in normal cells, may also be increased in these cells.

Example 8: OA-519 expression analyzed by *in situ* hybridization

cDNA from a ZR-75-1 library yielded a 1.6 kb probe, pFAS 1.6, showing -85% nucleotide identity with 3' sequences of rat fatty acid synthase cDNA. On Northern blots of total ZR-75-1 RNA, this probe hybridized with a single

~9.5 kb message (data not shown). *In situ* hybridization for OA-519 in formalin-fixed paraffin-embedded ZR-75-1 and DU-4475 human fibroblast cells using digoxigenin-labeled riboprobes derived from pFAS 1.6 in Bluescript II is shown in Figure 9. The left panel is anti-sense, while the right panel is the sense control. Anti-sense riboprobes generated from pFAS 1.6 yielded a substantially stronger hybridization signal with ZR-75-1 cells than with DU-4475 cells (Figure 9), showing that message levels and protein levels were concordant. Thus, cells that express OA-519 can be detected by either immunohistochemistry or *in situ* hybridization.

These data together suggest that OA-519 over-expression is from increased message levels, due either to increased transcriptional activation or to prolonged message stability. Increased OA-519 levels were not likely due to prolongation of OA-519 protein half-life since OA-519 protein over-expression was accompanied by OA-519 message over-expression. Similarly, experiments finding equivalent pFAS 1.6 hybridization signals among Southern blots of cell lines differing widely in OA-519 expression indicated that over-expression was not likely from gene amplification,

Example 9: Selective Expression of OA-519 in Human Breast Carcinoma

Figure 10 shows immunohistochemical staining with affinity purified anti-native OA-519 rabbit polyclonal antibodies on a formalin-fixed paraffin-embedded section of infiltrating duct carcinoma. OA-519 was detected using standard biotin-avidin immunohistochemistry with diaminobenzidine as the chromogen. The specificity of expression of FAS is demonstrated in Figure 10. The cytoplasm of the infiltrating breast cancer cells were strongly reactive with anti-OA-519 antibodies while the adjacent rim of histologically normal breast epithelial and stromal cells was unreactive. Note the intense brown staining in the cytoplasm of the tumors cells indicating OA-519 expression while the normal breast duct and lobule (arrows) and breast stoma were negative. This differential expression of fatty acid synthase between cancer and normal cells forms the basis of the potential selective *in vivo* inhibition of FAS.

Specific Inhibitors of FAS

The following examples show the effect of cerulenin on tumor cells. Cerulenin, a specific and non-competitive inhibitor of fatty acid synthase, was studied in tumor cells as a representative inhibitor of fatty acid synthesis. Cerulenin covalently binds to a thiol group in the active site of the condensing enzyme of fatty acid synthase, inactivating this key enzymatic step (Funabashi, et al., *J. Biochem.*, 105:751-755, 1989). The condensing enzyme reaction, which catalyzes the condensation of malonyl-CoA with an acetyl group or with the nascent fatty acid chain, generating $CO_2$, is the most specific reaction of the synthase and is not shared by other enzymes. While cerulenin has been noted to possess other activities, these either occur in microorganisms which may not be relevant models of human cells (e.g. inhibition of cholesterol synthesis in fungi, Omura (1976), *Bacteriol. Rev.,* 40:681-697; or diminished RNA synthesis in viruses, Perez, et al. (1991), *FEBS*, 280:129-133), occur at a substantially higher drug concentrations (inhibition of viral HIV protease at 5 mg/ml, Moelling, et al. (1990), *FEBS*, 261:373-377) or may be the direct result of the inhibition of endogenous fatty acid synthesis (inhibition of antigen processing in B lymphocytes and macrophages, Falo, et al. (1987), *J. Immunol.*, 139:3918-3923). Recent data suggests that cerulenin does not specifically inhibit myristoylation of proteins (Simon, et al., *J. Biol. Chem.*, 267:3922-3931, 1992). At the concentrations used in the present studies, cerulenin is a specific growth inhibitor for human tumor cells expressing significant FAS activity, as shown below.

Example 10: Cerulenin Cytotoxicity parallels Acyl-glyceride Synthesis in Cancer Cell Lines and Human Fibroblasts

Cerulenin cytotoxicity was studied in two experimental formats. First, clonogenic (limiting dilution) assays tested whether cerulenin was cytotoxic, cytostatic, or had no demonstrable effect. Once cytotoxicity was established, a high-throughput 96 well micro-titer plate assay was used to test various doses on most of the cell lines from Table 3. If cancer cells rely on endogenous fatty acid biosynthesis, it follows that the toxicity of cerulenin should be directly proportional to the level of fatty acid biosynthesis; i.e., cells with higher levels of FAS should be more sensitive to cerulenin.

Clonogenic assay: One million cells were plated in 25 cm sq. flasks. Following overnight incubation, cells were either untreated or treated with cerulenin at 2.5, 5.0 or 10.0 μg/ml for 6 hours. Cells were then trypsinized to a single cell suspension and plated in 16 mm dishes at 1000 or 500 cells per dish in triplicate. Following 3 days incubation for ZR-75-1 and MCF-7 cells, and 7 days for SKBR3 cells, colonies were counted and expressed as the percentage of the untreated control.

As shown in Figure 11A, treating MCF-7 cells with cerulenin at 10 μg/ml in clonogenic assays resulted in dose-dependent cytotoxicity. Increasing exposure time to 24 hours at 10 μg/ml resulted in greater than three logs of cell kill. Thus cerulenin was cytotoxic to cell lines undergoing endogenous fatty acid synthesis.

Measurement of growth inhibition: Cell lines were plated in 96 well microtiter plates at 5,000 cells per well. Following 18 hours growth, cerulenin (diluted in 10% DMSO) was added to the media to achieve 10μg/ml. Each time

point was performed in quadruplicate with empty well controls. After 24 hour exposure to cerulenin, cells were stained with crystal violet, solubilized in 1% SDS, and were read on a Molecular Diagnostics automated plate reader at 570 nm. Error bars represent standard error of the mean.

Using the microtiter plate cytotoxicity assay, Figure 11B demonstrates that, after 24 hours exposure to 10 μg/ml cerulenin, relative growth inhibition was directly related to the previously measured rate of fatty acid biosynthesis in all six cell lines. The MCF-7 adriamycin-resistant cell line (MCF-7a) was the only significant exception. Graphing growth inhibition versus FAS activity yielded a similar result (see Example 6, Figure 7). Thus, growth inhibition to cerulenin was largely predicted by the level of endogenous fatty acid biosynthesis or FAS activity. Importantly, this holds true for both cancer cells and normal embryonic fibroblasts. Therefore, the relationship of cerulenin growth inhibition and fatty acid biosynthesis was not a feature exclusive to transformed cells.

Example 11: Cerulenin Caused Dose-Dependent Inhibition of $^{14}C$-acetate Incorporation into Acyl-glycerides, While Cholesterol Synthesis Was Not Consistently Altered by Cerulenin

Measurement of endogenous fatty acid incorporation into acyl-glycerides and cholesterol: For each cell type, 200,000 cells were plated in triplicate for non-polar and polar lipid analysis for each drug dose in 24 well plates. Following overnight growth, cells were incubated for 6 hours with cerulenin (diluted in 10% DMSO) added to achieve 0, 2.5, 5.0 or 10.0 μg/ml. For the last two hours of cerulenin incubation, 1 μCi of $^{14}C$ acetate was added per well. $^{14}C$-labeled lipids were then extracted, chromatographed, and detected as described above. Error bars represent standard error of the mean.

Figure 12A demonstrates that incorporation of $^{14}C$-acetate into acyl-glycerides was consistently and significantly inhibited in a dose-related manner by cerulenin in six cell lines listed in Table 3. In contrast, Figure 12B shows that cholesterol synthesis was variably and marginally affected, ranging from mild inhibition in MCF-7 cells to stimulation in SKBR3 cells. These data suggest that cerulenin was not directly affecting enzymes of cholesterol biosynthesis. Furthermore, there was no relationship between the baseline level of cholesterol biosynthesis in these cells and cerulenin growth inhibition (data not shown). Thus cerulenin inhibited acyl-glyceride synthesis in cancer cell lines and fibroblasts with no significant or consistent effect on cholesterol biosynthesis.

Example 12: Cerulenin inhibition is not Correlated with Cell Proliferation

Since OA-$519_{FAS}$ expression in clinical breast cancers and some cell lines is independent of proliferation as measured by mitotic index (Kuhajda, et al., *N. Engl. J. Med.*, 321:636-641, 1989), proliferating cell nuclear antigen (PCNA) expression (data not shown) or correlation of FAS expression with cell cycle by flow cytometry (Shurbaji, et al., *Lab. Invest.*, 6869A, 1993), it would be expected that cerulenin anti-proliferative activity would be independent of tumor cell proliferation. Using the microtiter plate assay, doubling times for six cell lines were determined and compared to cerulenin growth inhibition (10 μg/ml, 24h). There was no correlation between tumor cell doubling times and cerulenin growth inhibition or FAS expression (Figure 13).

Example 13: Anti-proliferative Activity of Cerulenin Against Prostatic Carcinoma Cells

The antiproliferative activity of cerulenin was also extended to two human androgen-independent prostatic carcinoma lines that exhibited relatively high FAS activities (see Table 3). TSU-pr1 and Dupro-1 cell lines were plated in RPMI-1640 with 10% fetal bovine serum at 3 X $10^5$ and 1 X $10^5$ cells respectively/T25 flask in a humidified atmosphere of 95% air: 5% $CO_2$. After 24 hours, initial plating density was measured. In addition, media was replaced (control) or cerulenin was added at 2.5-10 μg/ml to duplicate cultures. Forty-eight hours later, cells were trypsinized and viable cells (trypan blue excluding) counted. The number of population doublings was estimated. For both cell lines, a 48-hour exposure to cerulenin (3-4 μg/ml) resulted in 50% inhibition of population doubling, relative to untreated cultures. Figure 14 shows representative data from one of two independent experiments.

Example 14: Growth Inhibition of Cancer Cell Lines by an Inhibitor of Acetyl CoA Carboxylase

TOFA (an inhibitor of acetyl CoA carboxylase) also demonstrated an anti-proliferative effect on a panel of cell lines with varying levels of FAS enzyme activity (shown in Table 3) and fatty acid biosynthesis (shown in Figure 8). This experiment was performed using the crystal violet growth inhibition assay. Cells with varying degrees of FAS activity were plated at 5000 cells per well in 96 well plates in RPMI-1640 with 10% heat-inactivated fetal bovine serum. After 72 hours of incubation in a humidified 95% air: 5% $CO_2$ atmosphere, varying concentrations (0-500 μg/ml) of TOFA (5-tetradecyloxyl)-2-furoic acid were added and cultures were re-incubated for 48 hours. Plates were then removed and stained with crystal violet as described. Absorbance readings were averaged for each concentration of

agent (n=4), control (n=8), and background (n=8). Background readings were subtracted. The concentration of TOFA is plotted against growth (as % of control growth) in Figure 15.

48-hour exposure to TOFA, an inhibitor of acetyl CoA carboxylase (Halvorson, D.L. and McCune, S.A., Lipids 19: 851-856, 1984) resulted in growth inhibition of the SKBR-3, ZR-75-1, SW480, and H'27 cell lines with ID50s of 4.6, 0.8, 59.8 and 44.7μg/ml, respectively. TOFA or 5-(tetradecyloxyl)-2-furoic acid, was a more potent inhibitor of the growth of high FAS cell lines (ZR-75-1 and SW80) than of those lines that expressed lower levels of FAS activity (HS27 and SK-8R-3), as shown in Figure 15.

Example 15: Inhibition of SKBR-3 Cell Growth by Various Inhibitors of Fatty Add Synthesis

In these experiments, cells were plated at 5000 cells per well in a 96-well microtiter plate and maintained in RPMI-1640 with 10% heat-inactivated fetal bovine serum for 72 hours. Next, the compounds indicated in Table 4 were added, and cells were incubated for an additional 48 hours. Growth inhibition was then measured using the crystal violet method.

The concentration of cerulenin required to inhibit the growth of the human SK-BR-3 mammary carcinoma line (shown in Table 3 to be a line with high FAS activity) was similar to that required for growth inhibition of the prostate tumor lines of Example X-4-A. As shown in Table 4, a 48 hour exposure to 3.6 μg/ml resulted in 50% inhibition of growth. Also shown in Table 4, other compounds reported to inhibit enzymes involved in fatty acid biosynthesis potently inhibited the growth of the SK-BR-3 mammary carcinoma line in a similar fashion, with 50% inhibitory doses (ID50) ranging from 0.3-61.4 μg/ml.

Taken together these data show that cerulenin as well as inhibitors of other enzymes of fatty acid synthesis potently inhibit the growth of mammary, colon, and prostatic carcinoma lines. Furthermore, the potency of growth inhibition was proportional to the relative levels of fatty acid biosynthesis exhibited by these cultured cells.

Table 4:

**Growth Inhibition of SK-BR-3 Cell Line by Inhibitors of Enzymes Involved In Endogenous Fatty Acid Biosynthesis**

| ENZYME TARGET | COMPOUND | $ID_{50}$ (μg/ml) |
|---|---|---|
| Citrate Lyase | S-carboxymethyl CoA | 61.4 |
| Malic Enzyme | Bromopyruvate | 52.0 |
| | Gossypol | 3.1 |
| | N-ethylmaleimide | 0.9 |
| Acetyl CoA Carboxylase | Sethoxydim | 9.5 |
| | 5-(tetradecyloxyl)-2-furoic acid | 4.6 |
| Fatty Acid Synthase | Cerulenin | 3.6 |
| | Iodoacetamide | 0.3 |

SK-BR-3 human mammary carcinoma cells, which were shown to have high levels of FAS activity were plated at 5000 cells/well in 96-well microtiter plates and maintained in RPMI-1640 with 10% heat-inactivated fetal bovine serum in a humidified atmosphere of 95% air: 5% $CO_2$. After 72 hours, compounds were added and cells were incubated for an additional 48 hours. Plates were stained with crystal violet and absorbances measured as described. The $ID_{50}$ represents the dose of compound which resulted in 50% reduction of control absorbance, as measured and analyzed in quadruplicate.

Example 16: Synergy Between Triacsin-C and Cerulenin

96 well microtiter plates were plated with ZR-75-1 cells at 25,000 cells/well and incubated overnight (18 h) at 37° C in 5% $CO_2$. Triacsin-C was added to achieve the following micromolar concentrations in the presence of 0, 2.5, 5.0 or 10.0 μg/ml cerulenin: 50, 25, 12.5, 6.2, 3.1, 1.6, 0.8, 0.4, 0.2, 0.1 and 0. Each concentration was performed in quadruplicate. Cells were incubated for 24 hours, washed in PBS, solubilized in 1% SDS, stained with 0.2% crystal violet in 2% ethanol, and read at 490 nm on a Molecular Diagnostics plate reader.

Figure 16 shows that triacsin-C is growth inhibitory to cells of a tumor cell line (ZR-75-1) that expresses $OA\text{-}519_{FAS}$. When cerulenin was added at a level approximating its $ID_{50}$, the $ID_{50}$ for triacsin-C was lowered from about 15 μM to less than 1 μM, demonstrating the synergistic effect exhibited by the combination of these two inhibitors.

Example 17: High Concentrations of Exogenous Oleate Rescues ZR-75-1 Cells from Growth Inhibition by Cerulenin

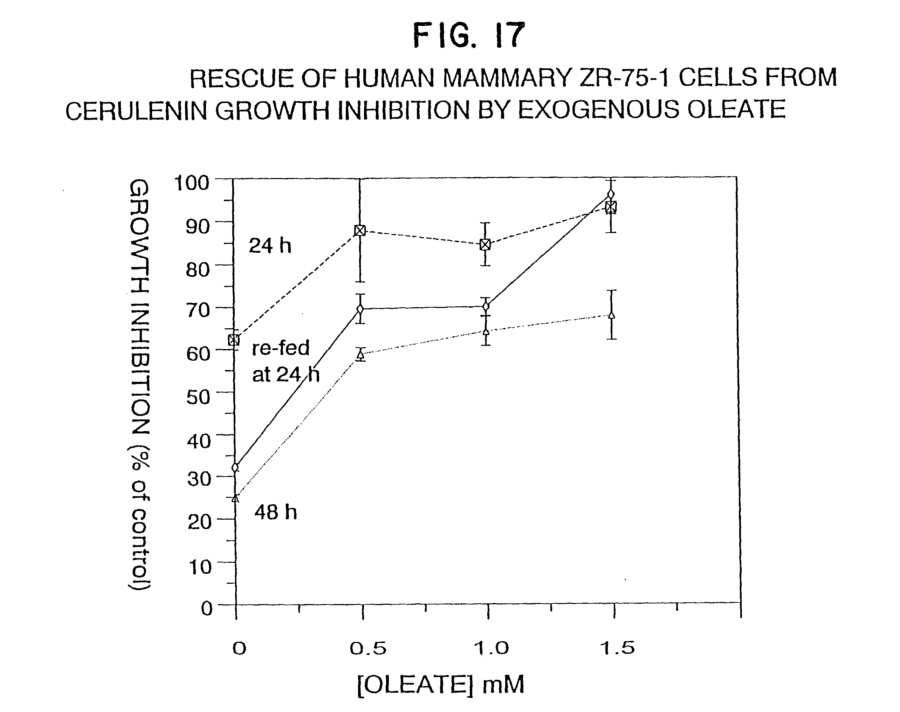

Figure 17 demonstrates that cerulenin inhibition of in ZR-75-1 cell growth is reversible with high concentrations of exogenous oleate. ZR-75-1 cells (5,000 per well) were plated in 96 well microtiter plates in medium supplemented with 0, 0.5, 1.0 or 1.5 mM oleate bound to delipidized bovine serum albumin at a molar ration of 4:1. After 18 hours incubation, cerulenin was added at a dose of 10 µg/ml. Cells were stained and assayed as in Example 10 after 24 hours (Figure 17, upper line) or 48 hours (lower line). The middle line of Figure 17 represents cells which were re-fed at 24 hours with medium containing the respective oleate concentration without cerulenin and assayed after a 48 hour total incubation. Error bars represent standard error of the mean.

In the top curve of Figure 17, where cells were exposed to 10 µg/ml cerulenin for 24 hours in the presence of 0, 0.5, 1.0 or 1.5 mM oleate, the 1.5 mM concentration of oleate achieved virtually complete reversal of growth inhibition by cerulenin. When the cells are allowed to incubate for 48 hours, significant toxicity occurred as demonstrated by the lower curve, which was not reversed by oleate. If the anti-proliferative activity seen at 48 hours were due to depletion of oleate in the medium, re-feeding the cells at 24 hours with fresh medium containing the same respective oleate concentration, but without additional cerulenin, should rescue the cells. The middle curve shows the effect of re-feeding the cells at 24 hours, which indeed rescued the cells after 48 hours with 1.5 mM oleate. When oleate was absent, the refed and nonrefed cells showed similar cerulenin-mediated growth inhibition.

This experiment demonstrates that rescue from the anti-proliferative activity of cerulenin was due to the additional fatty acid, and not to glucose or other substances such as growth factors present in the medium. Interestingly, cell lines with low FAS activity such as SW-480 and normal human fibroblasts, required a lower (0.5 mM), physiologic concentration of oleate to achieve rescue from cerulenin growth inhibition (see Example 5). The level of oleate required to rescue ZR-75-1 cells (1.5 mM) is superphysiological, and it is unlikely that this level could be achieved by a diet containing elevated fatty acid content. Taken together these data suggest that cerulenin acts by creating a state of fatty acid starvation leading to cell death, which is proportional to the level of endogenous fatty acid synthesis.

## Claims

1. The use of an inhibitor of fatty acid synthesis in the preparation of a pharmaceutical composition for inhibiting, in a human, growth of tumour cells expressing at least one enzyme of the fatty acid biosynthetic pathway, **characterised in that** the inhibitor specifically inhibits fatty acid biosynthesis by a cell as demonstrated by inhibition of growth *in vitro* of a tumour cell expressing FAS, said inhibition of growth *in vitro* being reversed by oleate, said inhibitor being specifically cytotoxic to said tumour cells.

2. The use according to claim 1, wherein said tumour cells express a protein exhibiting fatty acid synthase activity.

3. The use according to any one of claims 1-2, wherein said patient has a carcinoma selected from the group consisting of breast carcinoma, rectal carcinoma, ovarian carcinoma, colon carcinoma and prostate carcinoma.

4. The use according to any of claims 1-3, further wherein fatty acid synthase expression of the patient's normal tissue is down-regulated during treatment.

5. The use according to claim 4, wherein down-regulation of fatty acid synthase expression is accomplished by reducing the caloric intake of said patient.

6. The use according to claim 4, wherein down-regulation of fatty acid synthase expression is accomplished by administering a composition containing long chain free fatty acid or acyl glyceride to said patent.

7. The use according to claim 6, wherein the composition containing long chain free fatty acid or acyl glyceride supplies essential fatty acids to said patient.

8. The use according to any of claims 1-7, wherein the inhibitor of fatty acid synthesis is an inhibitor of an enzyme selected from the group consisting of fatty acid synthase, acetyl CoA carboxylase, citrate lyase and malic enzyme.

9. The use according to claim 8, wherein the enzyme is fatty acid synthase (FAS).

10. The use according to claim 9, wherein the inhibitor is cerulenin.

**11.** The use according to claim 8, wherein the enzyme is acetyl CoA carboxylase.

**12.** The use according to claim 11, wherein the inhibitor is 5-(tetradecyloxy)-2-furoic acid (TOFA).

**13.** The use according to any one of claims 1-12, **characterised in that** treatment further comprises administration of an inhibitor of lipid biosynthesis substantially contemporaneously with said pharmaceutical composition.

**14.** The use according to claim 13, wherein the inhibitor of lipid biosynthesis is Triacsin C.

**15.** An assay method to aid in determining virulence of tumour cells in a patient, comprising:

a. obtaining a sample from said patient;
b. determining the amount of a protein exhibiting fatty acid synthase activity in said sample, wherein said step of determining further comprises detecting binding between said protein and an antibody which immunologically binds said protein, wherein said antibody is not immunologically cross-reactive with haptoglobin 1 or 2 or with a polypeptide having the amino acid sequence of leu tyr ser gly asn asp val thr asp ile ser asp asp arg phe pro lys pro pro glu ile ala asn gly tyr val glu lys leu phe arg tyr gln cys, expression by tumour cells of a protein exhibiting fatty acid synthase activity being indicative of virulence.

**16.** An assay method to aid in determining virulence of tumour cells in a patient, comprising:

a. obtaining a sample from said patient;
b. determining the amount of a protein exhibiting fatty acid synthase activity in said sample, wherein said step of determining comprises detecting hybridisation between a nucleic acid probe and mRNA encoding said protein, expression by tumour cells of a protein exhibiting fatty acid synthase activity being indicative of virulence.

**17.** The method according to claim 15 or claim 16, wherein said sample is a sample of tumour tissue.

**18.** The method according to claim 15 or claim 16, wherein said sample is biological fluid from said patient.

**19.** The method according to any one of claims 15-18, wherein the tumour cells are from a breast carcinoma, prostate carcinoma, or ovarian carcinoma.

**Patentansprüche**

**1.** Verwendung eines Inhibitors der Fettsäuresynthese bei der Herstellung einer pharmazeutischen Zusammensetzung zur Hemmung, beim Menschen, des Wachstums von Tumorzellen, die wenigstens ein Enzym des Fettsäure-Biosyntheseweges exprimieren, **dadurch gekennzeichnet, daß** der Inhibitor spezifisch die Fettsäurebiosynthese durch eine Zelle hemmt, wie durch Wachstumshemmung einer FAS exprimierenden Tumorzelle in vitro gezeigt, wobei die Wachstumshemmung in vitro durch Oleat aufgehoben wird, wobei der Inhibitor spezifisch cytotoxisch für die Tumorzellen ist.

**2.** Verwendung nach Anspruch 1, wobei die Tumorzellen ein Protein exprimieren, das Fettsäuresynthase-Aktivität zeigt.

**3.** Verwendung nach irgendeinem der Ansprüche 1-2, wobei der Patient ein Karzinom hat, das ausgewählt ist aus der Gruppe bestehend aus Brustkarzinomen, Rektalkarzinomen, Ovarialkarzinomen, Darmkarzinomen und Prostatakarzinomen.

**4.** Verwendung nach irgendeinem der Ansprüche 1-3, wobei ferner die Fettsäuresynthase-Expression des normalen Gewebes des Patienten während der Behandlung herunterreguliert wird.

**5.** Verwendung nach Anspruch 4, wobei die Herunterregulierung der Fettsäuresynthase-Expression durch Reduktion der Kalorienaufnahme des Patienten erreicht wird.

**6.** Verwendung nach Anspruch 4, wobei die Herunterregulierung der Fettsäuresynthase-Expression durch Verabreichung einer Zusammensetzung an den Patienten erreicht wird, die langkettige freie Fettsäure oder Acylglycerid umfaßt.

**7.** Verwendung nach Anspruch 6, wobei die Zusammensetzung, die langkettige Fettsäure oder Acylglycerid enthält, dem Patienten essentielle Fettsäuren zuführt.

**8.** Verwendung nach irgendeinem der Ansprüche 1-7, wobei der Inhibitor der Fettsäuresynthese ein Inhibitor eines Enzyms ist, das aus der Gruppe ausgewählt ist, die aus Fettsäuresynthase, Acetyl-CoA-Carboxylase, Citratlyase und Malatenzym besteht.

**9.** Verwendung nach Anspruch 8, wobei das Enzym Fettsäuresynthase (FAS) ist.

**10.** Verwendung nach Anspruch 9, wobei der Inhibitor Cerulenin ist.

**11.** Verwendung nach Anspruch 8, wobei das Enzym Acetyl-CoA-Carboxylase ist.

**12.** Verwendung nach Anspruch 9, wobei der Inhibitor 5-(Tetradecycloxy)-2-furancarbonsäure (TOFA) ist.

**13.** Verwendung nach irgendeinem der Ansprüche 1-12, **dadurch gekennzeichnet, daß** die Behandlung ferner im wesentlichen gleichzeitig mit der pharmazeutischen Zusammensetzung die Verabreichung eines Inhibitors der Lipidbiosynthese umfaßt.

**14.** Verwendung nach Anspruch 13, wobei der Inhibitor der Lipidbiosynthese Triacsin C ist.

**15.** Assayverfahren zur Unterstützung bei der Bestimmung der Virulenz von Tumorzellen in einem Patienten, umfassend:

a. Erhalten einer Probe von dem Patienten;

b. Bestimmen der Menge eines Proteins mit Fettsäuresynthase-Aktivität in der Probe, wobei der Bestimmungsschritt ferner den Nachweis der Bindung zwischen dem Protein und einem Antikörper umfaßt, der das Protein immunologisch bindet, wobei der Antikörper nicht immunologisch kreuzreaktiv mit Haptoglobin 1 oder 2 oder mit einem Polypeptid mit der Aminosäuresequenz Leu Tyr Ser Gly Asn Asp Val Thr Asp Ile Ser Asp Asp Arg Phe Pro Lys Pro Pro Glu Ile Ala Asn Gly Tyr Val Glu Lys Leu Phe Arg Tyr Gln Cys ist, wobei die Expression eines Proteins mit Fettsäuresynthase-Aktivität durch Tumorzellen Virulenz anzeigt.

**16.** Assayverfahren zur Unterstützung bei der Bestimmung der Virulenz von Tumorzellen in einem Patienten, umfassend:

a. Erhalten einer Probe von dem Patienten;

b. Bestimmen der Menge eines Proteins mit Fettsäuresynthase-Aktivität in der Probe, wobei der Bestimmungsschritt den Nachweis der Hybridisierung zwischen einer Nukleinsäuresonde und einer für das Protein codierenden mRNA umfaßt, wobei die Expression eines Proteins mit Fettsäuresynthase-Aktivität durch Tumorzellen Virulenz anzeigt.

**17.** Verfahren nach Anspruch 15 oder 16, wobei die Probe eine Tumorgewebeprobe ist.

**18.** Verfahren nach Anspruch 15 oder 16, wobei die Probe eine biologische Flüssigkeit des Patienten ist.

**19.** Verfahren nach irgendeinem der Ansprüche 15-18, worin die Tumorzellen von einem Brustkarzinom, Prostatakarzinom oder Ovarialkarzinom sind.

## Revendications

**1.** Utilisation d'un inhibiteur de la synthèse d'acides gras dans la préparation d'une composition pharmaceutique pour inhiber, chez un humain, la croissance de cellules tumorales exprimant au moins une enzyme de la voie de biosynthèse des acides gras, **caractérisée en ce que** l'inhibiteur inhibe spécifiquement la biosynthèse d'acides gras par une cellule comme le démontre l'inhibition *in vitro* de la croissance d'une cellule tumorale exprimant une FAS, ladite inhibition *in vitro* de la croissance étant inversée par l'oléate, ledit inhibiteur étant spécifiquement cytotoxique pour lesdites cellules tumorales.

**2.** Utilisation selon la revendication 1, dans laquelle lesdites cellules tumorales expriment une protéine présentant une activité de synthase des acides gras.

**3.** Utilisation selon l'une quelconque des revendications 1 à 2, dans laquelle ledit patient a un carcinome sélectionné dans le groupe composé des carcinomes du sein, du rectum, de l'ovaire, du côlon et de la prostate.

**4.** Utilisation selon l'une quelconque des revendications 1 à 3, dans laquelle en outre l'expression d'une synthase des acides gras du tissu normal d'un patient est régulée en baisse pendant le traitement.

**5.** Utilisation selon la revendication 4, dans laquelle la régulation en baisse de l'expression d'une synthase des acides gras est accomplie en réduisant l'absorption calorique dudit patient.

**6.** Utilisation selon la revendication 4, dans laquelle la régulation en baisse de l'expression d'une synthase des acides gras est accomplie en administrant audit patient une composition contenant des acides gras ou glycérides d'acyle libres à longue chaîne.

**7.** Utilisation selon la revendication 6, dans laquelle la composition contenant des acides gras ou glycérides d'acyle libres à longue chaîne fournit les acides gras essentiels audit patient.

**8.** Utilisation selon l'une quelconque des revendications 1 à 7, dans laquelle l'inhibiteur de la synthèse d'acides gras est un inhibiteur d'une enzyme sélectionnée dans le groupe composé de la synthase des acides gras, acétyl CoA carboxylase, citrate lyase et enzyme malique.

**9.** Utilisation selon la revendication 8, dans laquelle l'enzyme est la synthase des acides gras (FAS).

**10.** Utilisation selon la revendication 9, dans laquelle l'inhibiteur est la cérulénine.

**11.** Utilisation selon la revendication 8, dans laquelle l'enzyme est l'acétyl CoA carboxylase.

**12.** Utilisation selon la revendication 11, dans laquelle l'inhibiteur est l'acide 5-(tétradécyloxy)-2-furoïque (TOFA).

**13.** Utilisation selon l'une quelconque des revendications 1 à 12, **caractérisée en ce que** le traitement comprend en outre l'administration d'un inhibiteur de la biosynthèse des lipides de manière substantiellement concomitante avec ladite composition pharmaceutique.

**14.** Utilisation selon la revendication 13, dans laquelle l'inhibiteur de la biosynthèse des lipides est la Triacsine C.

**15.** Procédé de dosage pour aider à la détermination de la virulence des cellules tumorales chez un patient, comprenant:

a. l'obtention d'un échantillon du patient;
b. la détermination de la quantité d'une protéine présentant une activité de synthase des acides gras dans ledit échantillon, dans laquelle ladite étape de détermination comprend en outre la détection d'une liaison entre ladite protéine et un anticorps qui se lie d'un point de vue immunologique à ladite protéine, dans laquelle ledit anticorps n'effectue pas de réaction croisée avec l'haptoglobine 1 ou 2 ou avec un polypeptide ayant la séquence d'acides aminés leu tyr ser gly asn asp val thr asp ile ser asp asp arg phe pro lys pro pro glu ile ala asn gly tyr val glu lys leu phe arg tyr gln cys, expression par les cellules tumorales d'une protéine présentant une activité de synthase des acides gras étant indicatrice de la virulence.

**16.** Procédé de dosage pour aider à la détermination de la virulence des cellules tumorales chez un patient, comprenant:

a. l'obtention d'un échantillon dudit patient;

b. la détermination de la quantité d'une protéine présentant une activité de synthase des acides gras dans ledit échantillon, dans laquelle ladite étape de détermination comprend la détection d'une hybridation entre une sonde d'acides nucléiques et un ARNm codant pour ladite protéine, expression par des cellules tumorales d'une protéine présentant une activité de synthase des acides gras étant indicatrice de la virulence.

**17.** Procédé, selon les revendications 15 ou 16, dans lequel ledit échantillon est un échantillon de tissu tumoral.

**18.** Procédé, selon les revendications 15 ou 16, dans lequel ledit échantillon est un fluide biologique issu dudit patient.

**19.** Procédé, selon l'une quelconque des revendications 15 à 18, dans lequel les cellules tumorales sont issues d'un carcinome du sein, de la prostate ou de l'ovaire.

FIG. 1

OA-519 (FAS) Expression and Survival of Breast Carcinoma

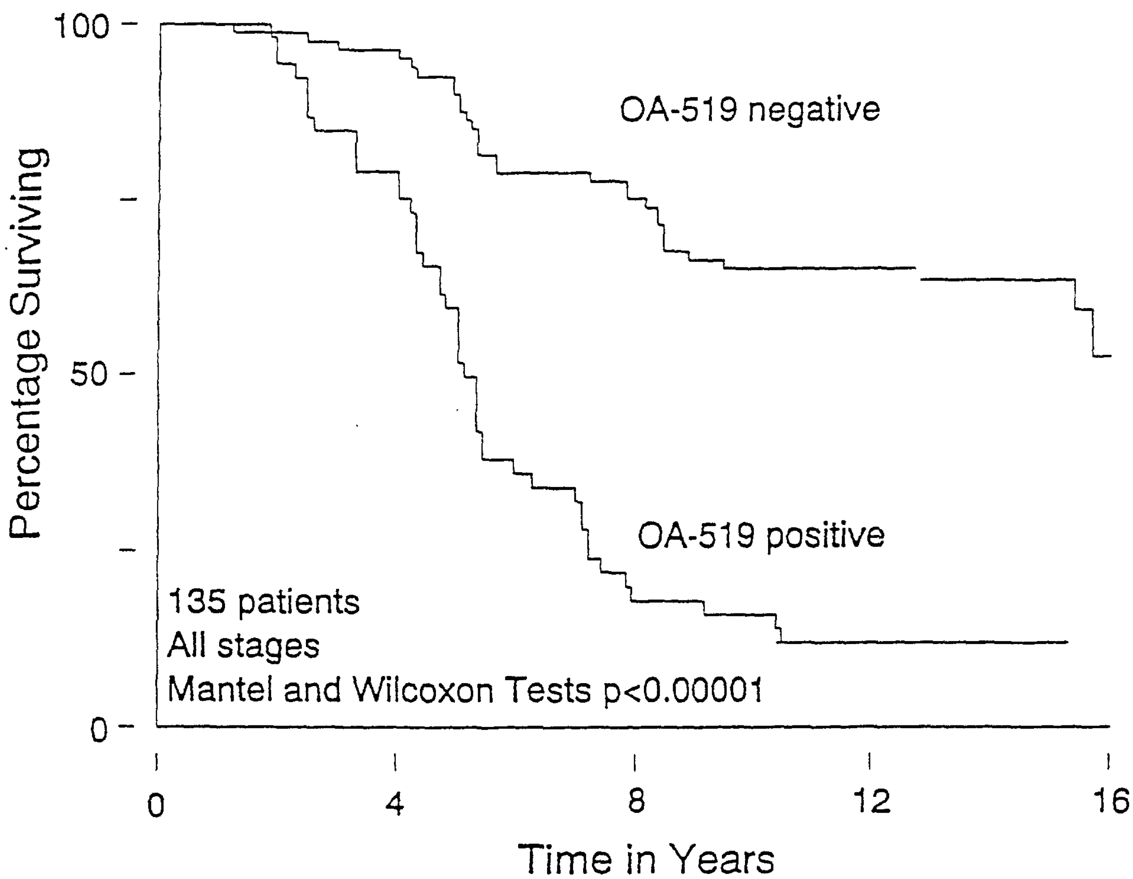

FIG. 1A

Immunohistochemical Detection of OA-519 and Progesterone Receptor are Prognostic for Breast Cancer Patients

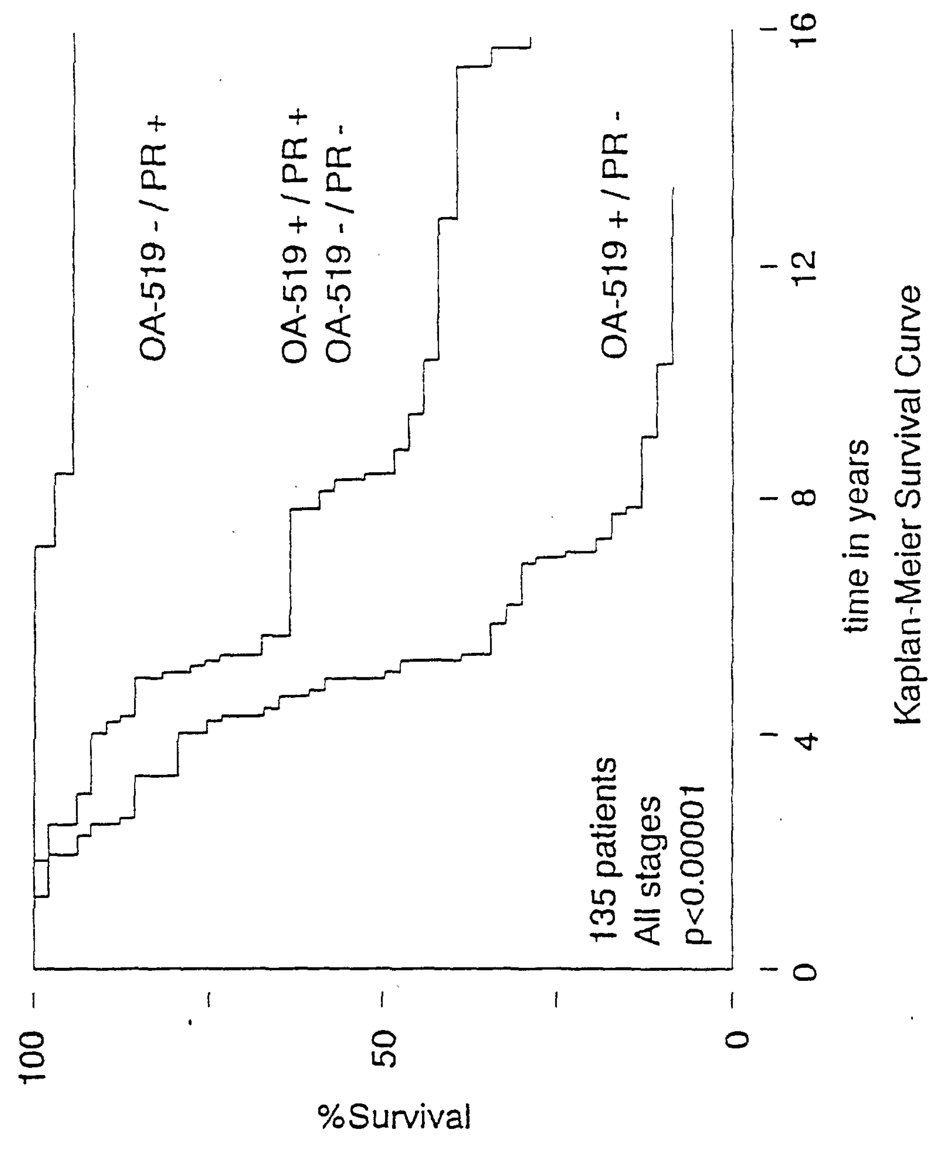

FIG. 2A

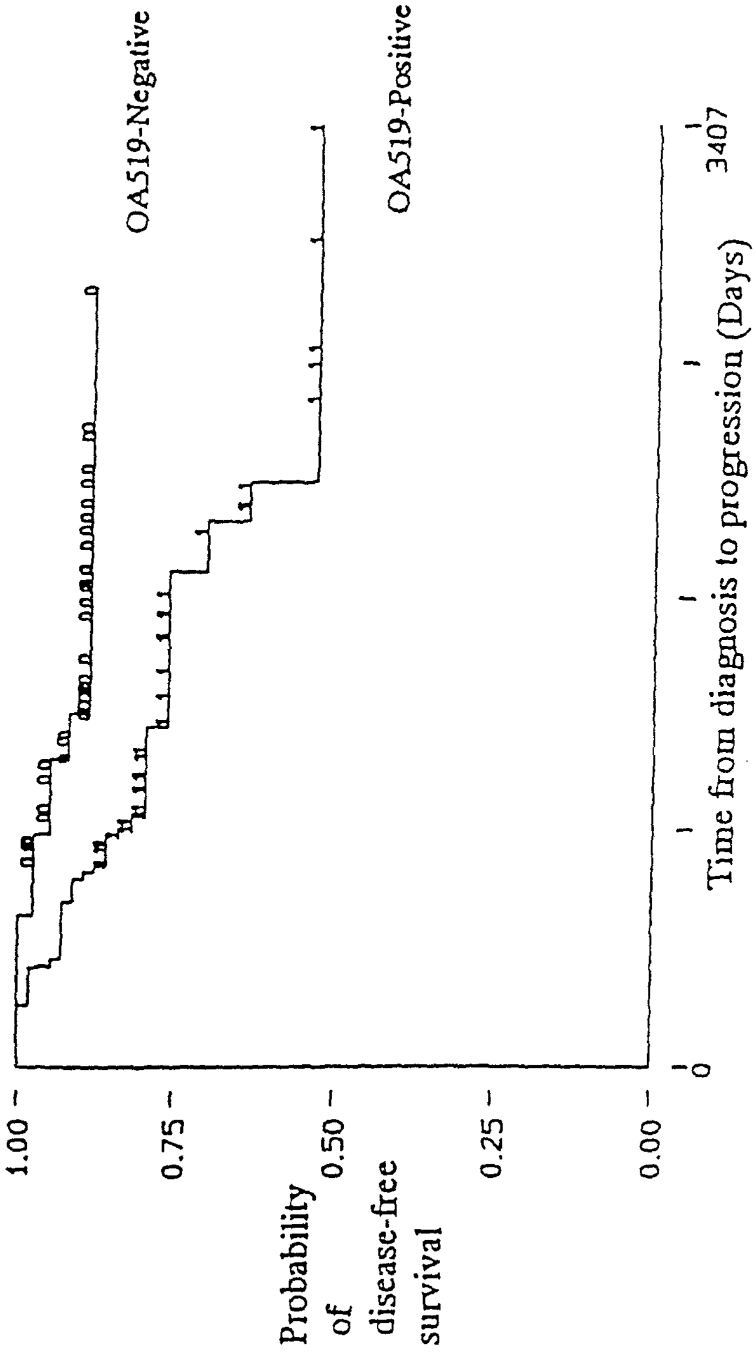
OA519-Negative
OA519-Positive
1.00
0.75
0.50
0.25
0.00
Probability of disease-free survival
0
3407
Time from diagnosis to progression (Days)

# FIG. 2B

OA-519 EXPRESSION AND PROGNOSIS IN OVARIAN CARCINOMA

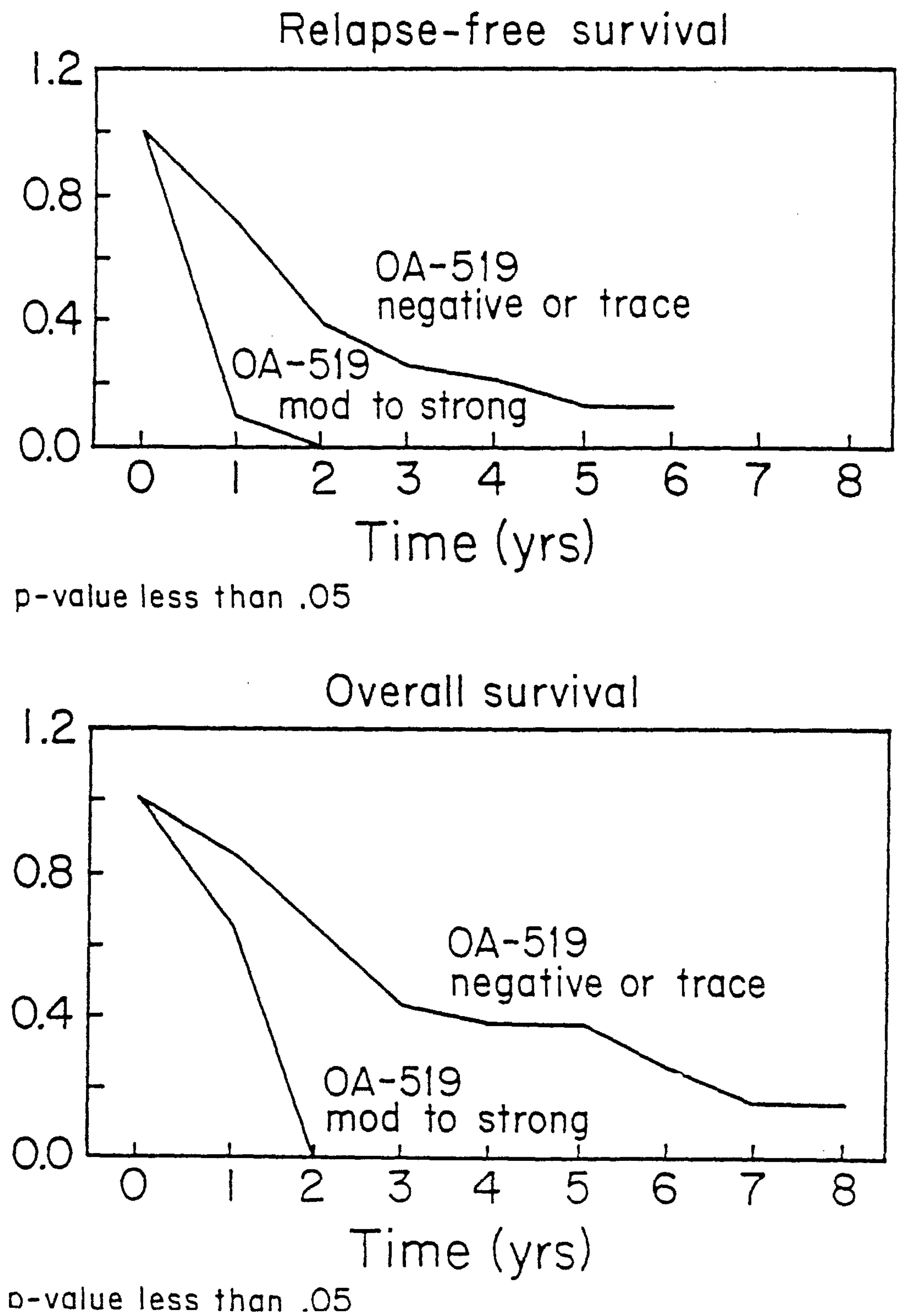

FIG. 2C

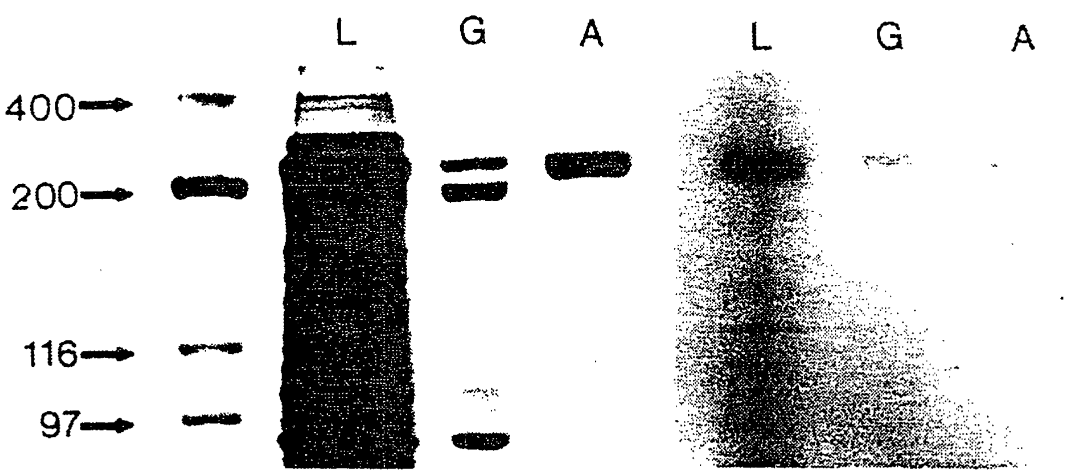

# FIG. 3A

34-Amino Acid Sequence of a Peptide
Immunologically Cross-Reactive with OA-519

leu tyr ser gly asn asp val thr asp ile ser asp asp arg phe pro lys pro pro glu ile ala asn gly tyr val glu lys leu phe arg tyr gln cys.

# FIG. 3B

## OA-519 Peptide Sequence Analysis

Sequence 1: Analysis of 134 kD OA-519 peptide sequence homology.

```
OA-519 peptide sequence:                 LQQHDVAQEQWXP
                                         |||||||||:||:|
Rat fatty acid synthase (EC 2.3.1.85): TKLQQHDVAQGQWDPSGPAPTNLGALD
                                             1290      1300
```

84.6% identity in 13 amino acid overlap.

Sequence 2: Analysis of OA-519 peptide sequence from Example 12 of the Continuation-In-Part of U.S. Serial No. 07/735522 filed July 26, 1991.

```
OA-519 peptide sequence:               HAVVLE
                                       ||||||
Rat fatty acid synthase (EC 2.3.1.85): HAVVLE
```

100% identity in 6 amino acid overlap.

FIG. 4

OA-519 Synthesizes Fatty Acids from Acetyl- and Malonyl-CoA

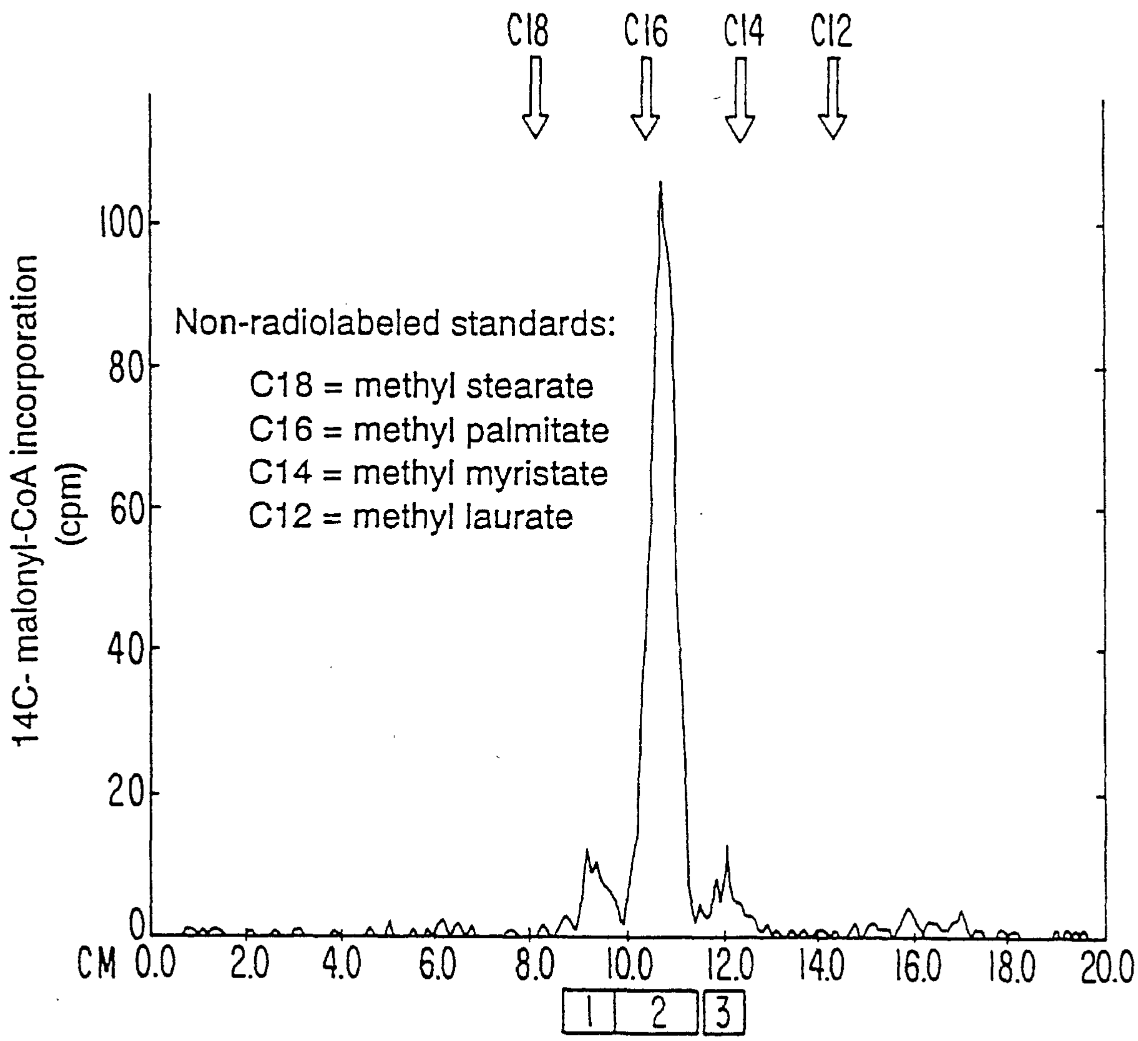

# FIG. 5

Dixon Plot of Cerulenin
Inhibition of OA-519 Fatty Acid Synthase Activity

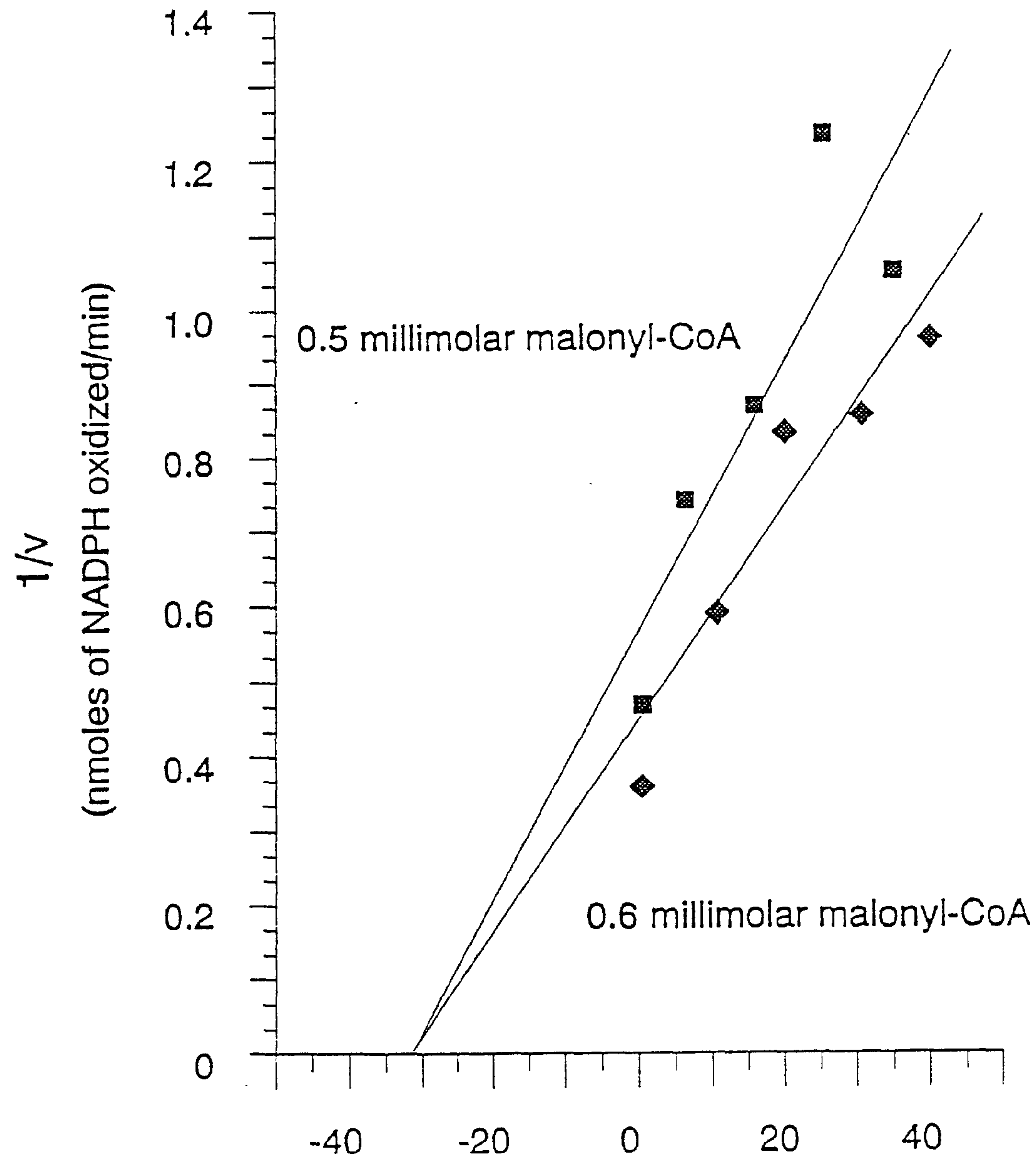

FIG. 6

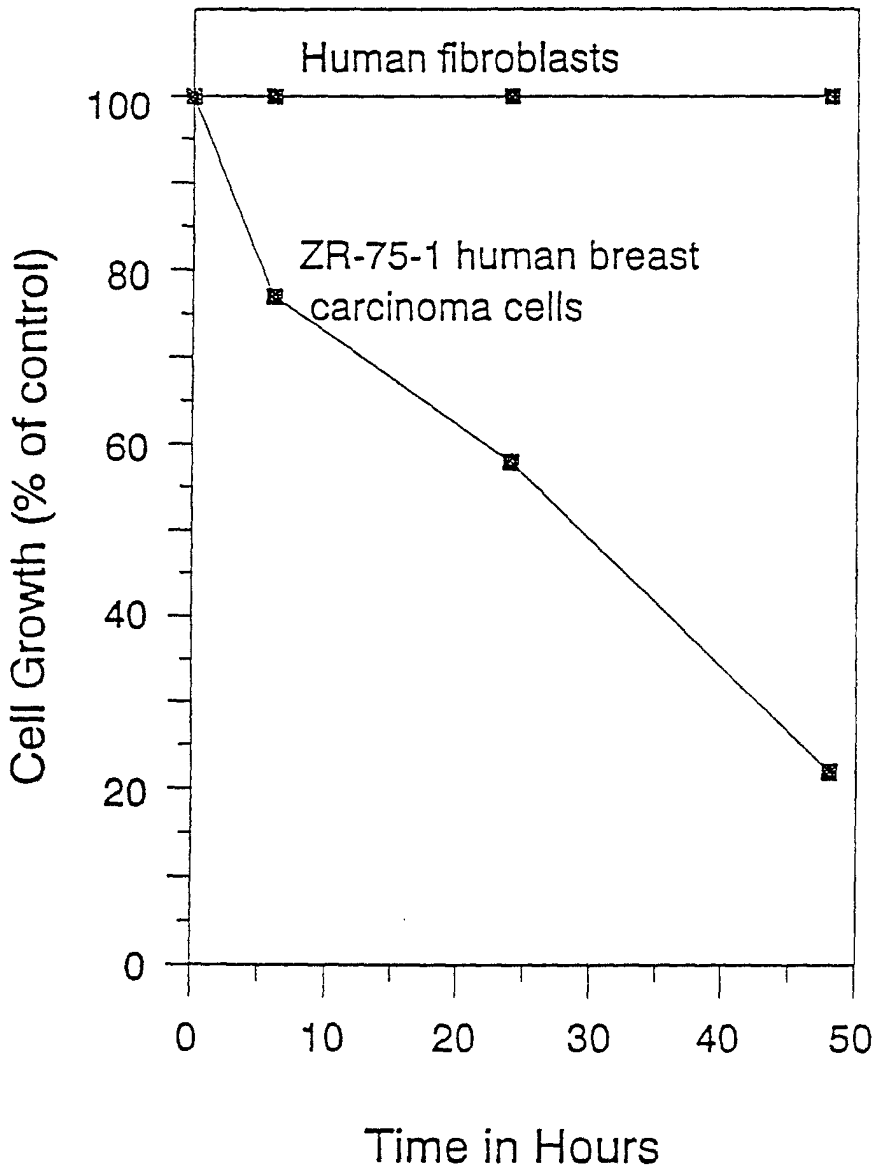
Human fibroblasts
ZR-75-1 human breast carcinoma cells
Cell Growth (% of control)
100
80
60
40
20
0
0
10
20
30
40
50
Time in Hours

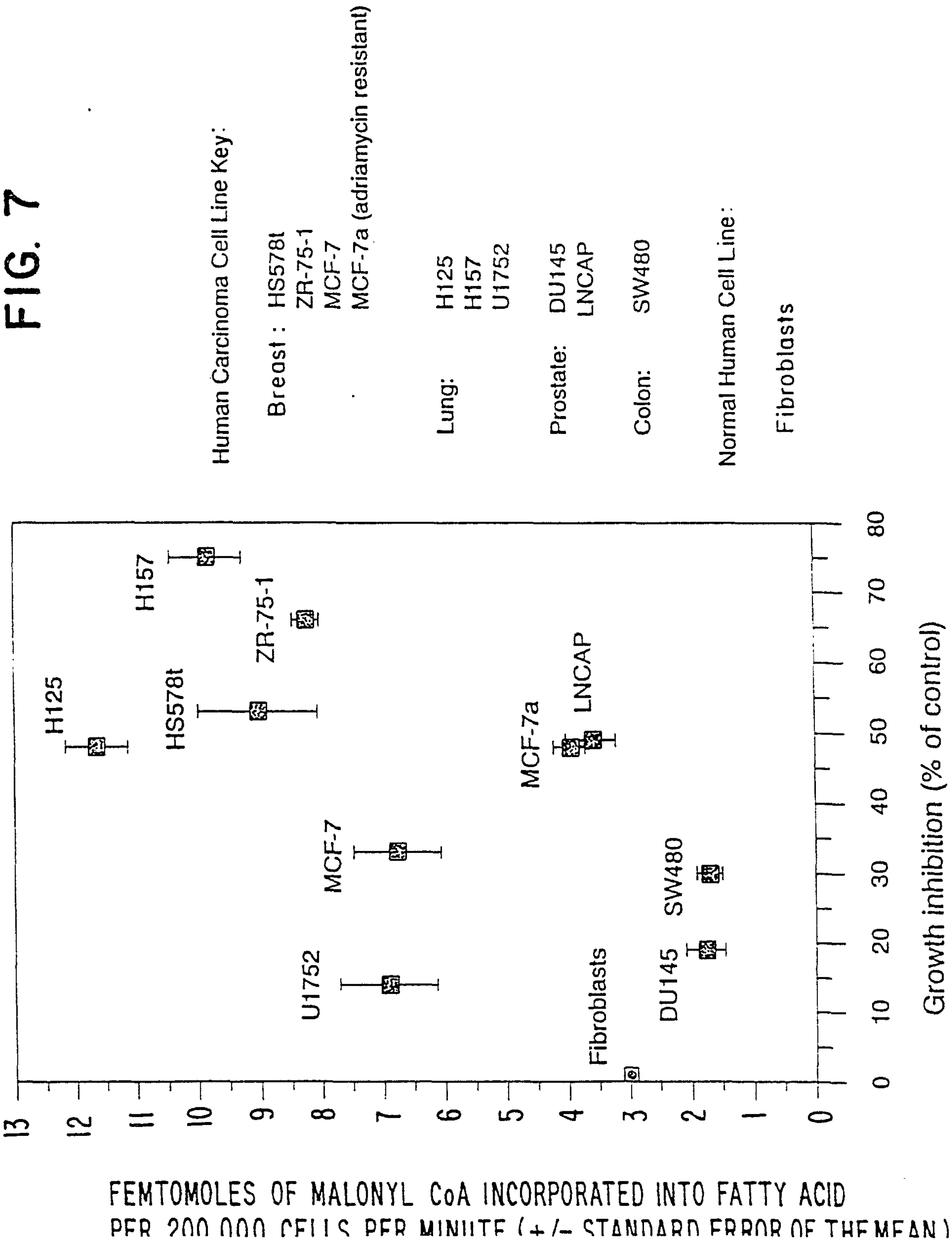
Human Carcinoma Cell Line Key:
Breast: HS578t
ZR-75-1
MCF-7
MCF-7a (adriamycin resistant)
Lung: H125
H157
U1752
Prostate: DU145
LNCAP
Colon: SW480
Normal Human Cell Line:
Fibroblasts
H125
H157
HS578t
ZR-75-1
MCF-7
MCF-7a
LNCAP
U1752
SW480
DU145
Fibroblasts
13
12
11
10
9
8
7
6
5
4
3
2
1
0
10
20
30
40
50
60
70
80
Growth inhibition (% of control)
FEMTOMOLES OF MALONYL CoA INCORPORATED INTO FATTY ACID
PER 200,000 CELLS PER MINUTE (+/- STANDARD ERROR OF THE MEAN)

FIG. 7

FIG. 8

Correlation of Fatty Acid Synthase (FAS) Activity and Total Fatty Acid Biosynthesis

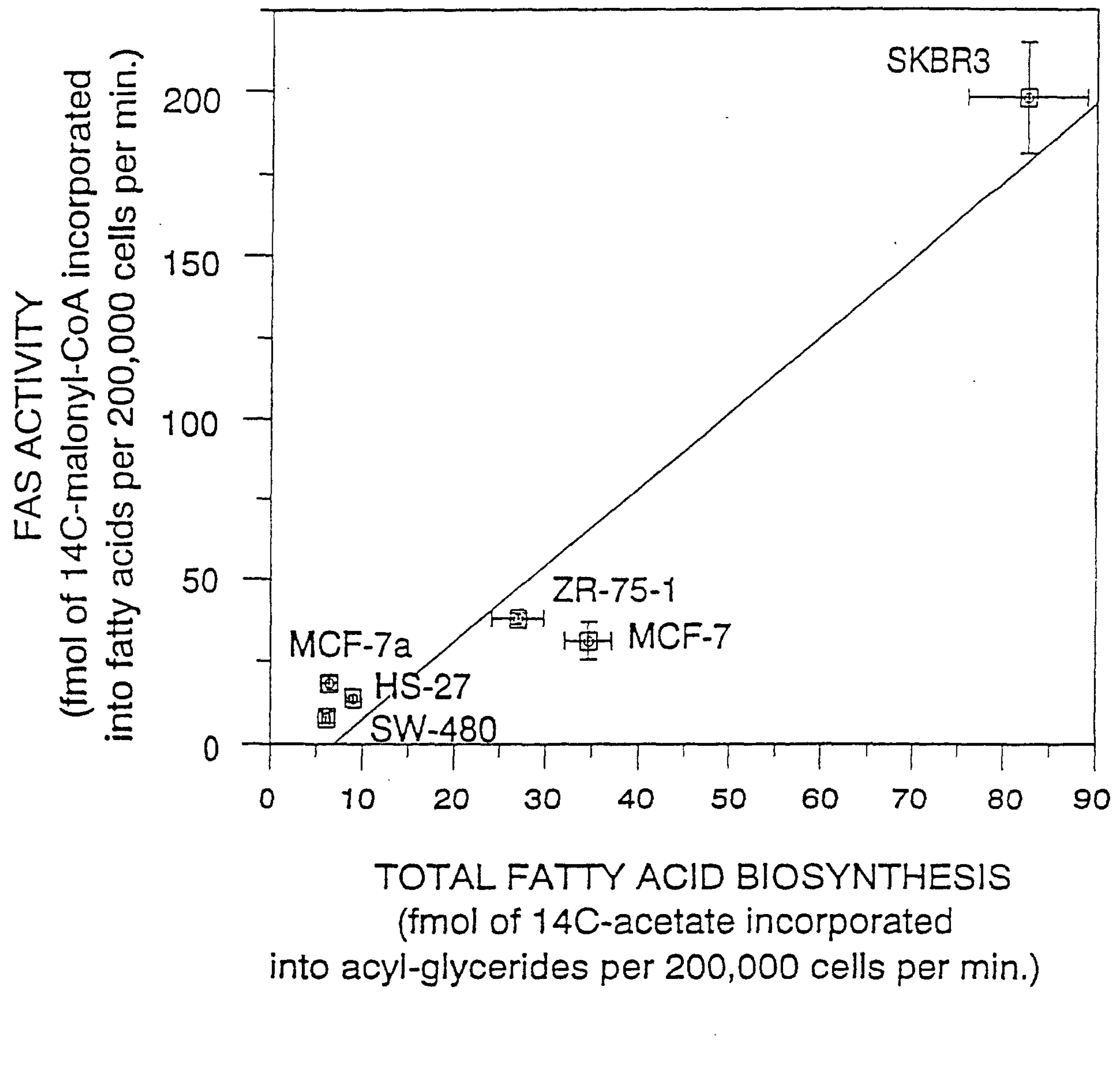

FIG. 9

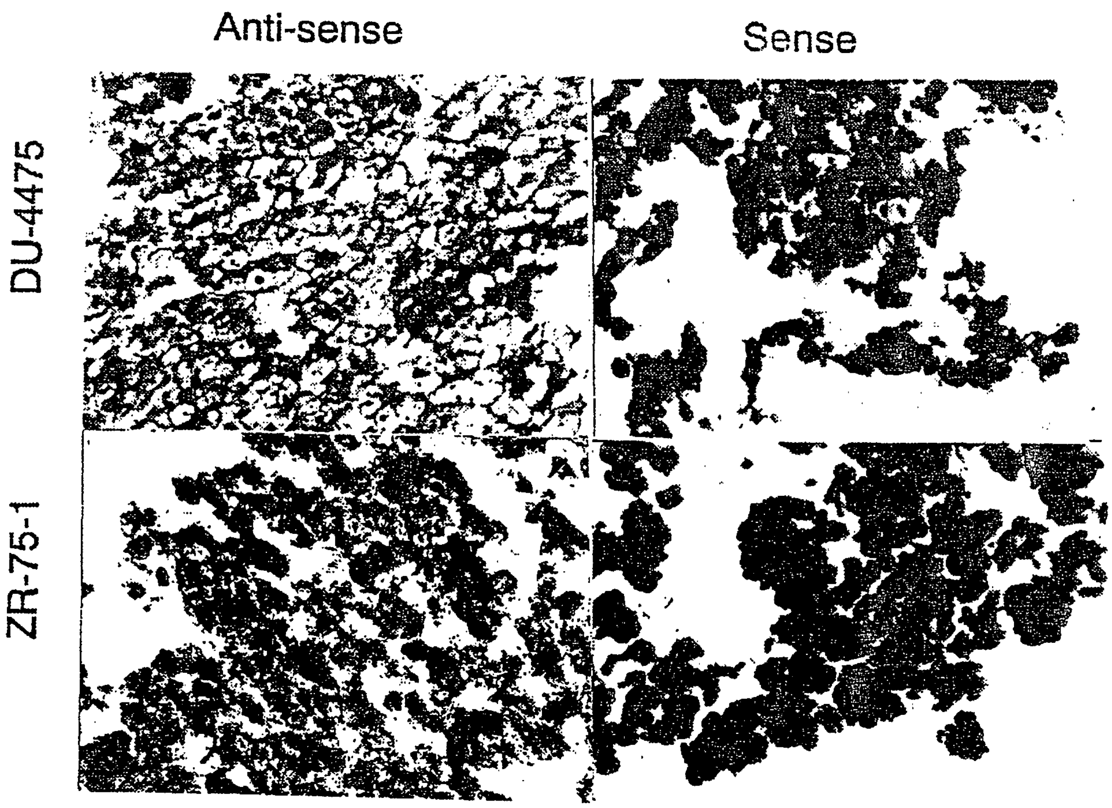

FIG. 10

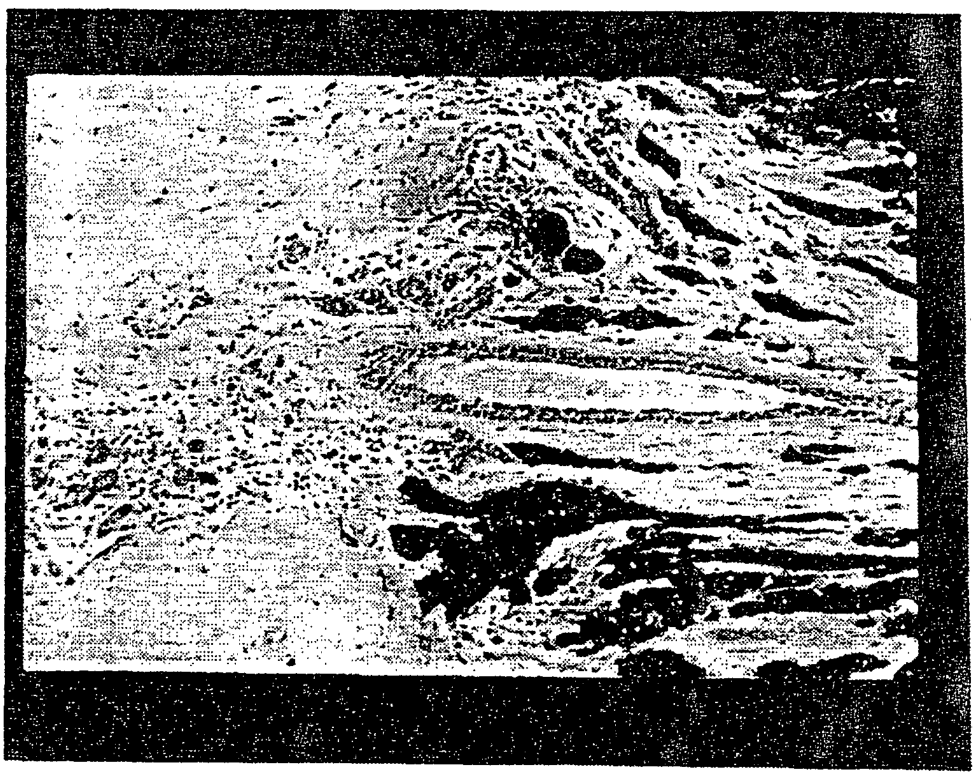

# FIG. 11A

Effect of Cerulenin on Clonogenic Survival of Human Mammary MCF-7 Cells

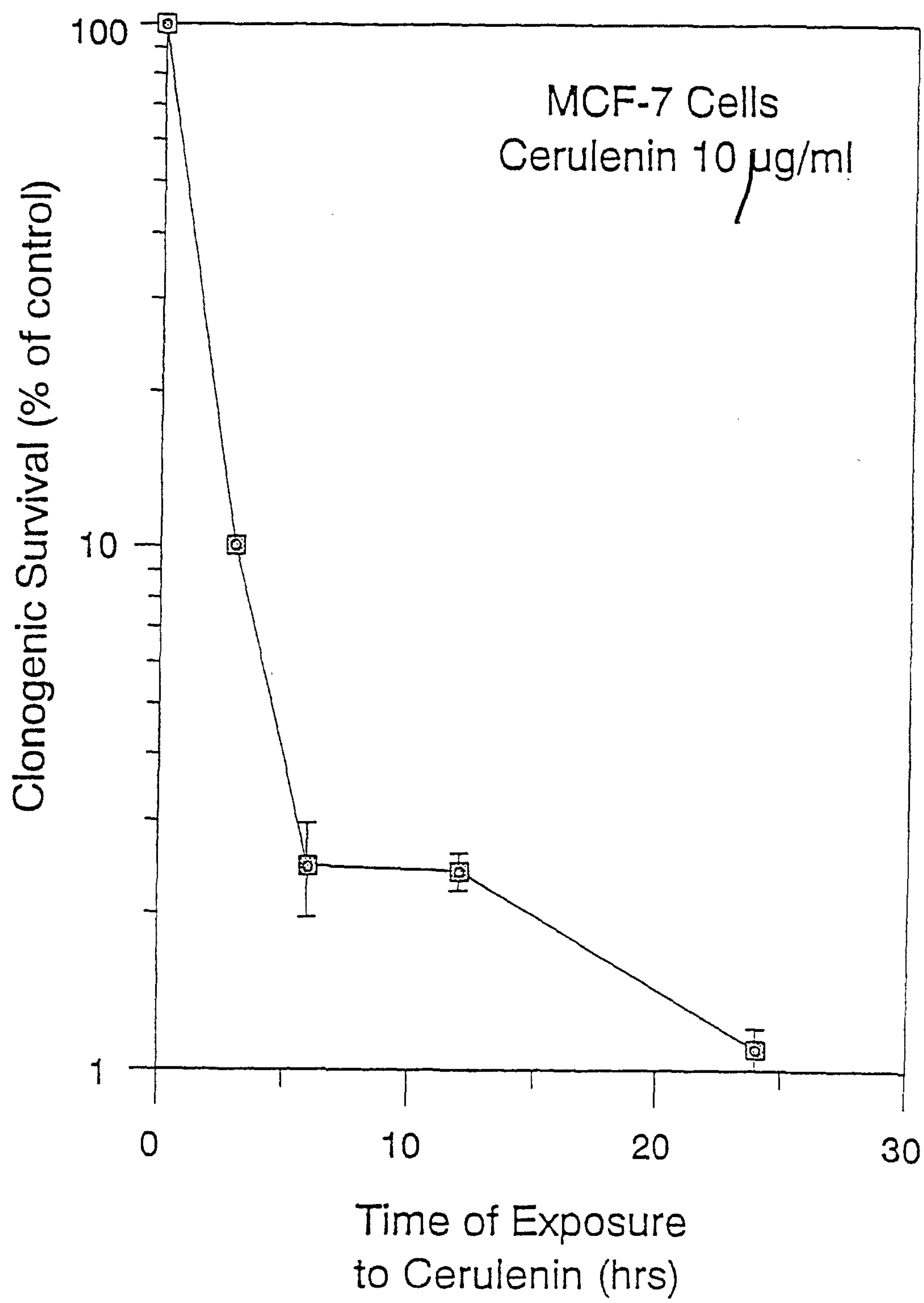

FIG. IIB

Correlation Between FAS Activity and Degree of Cell Growth Inhibition by Cerulenin

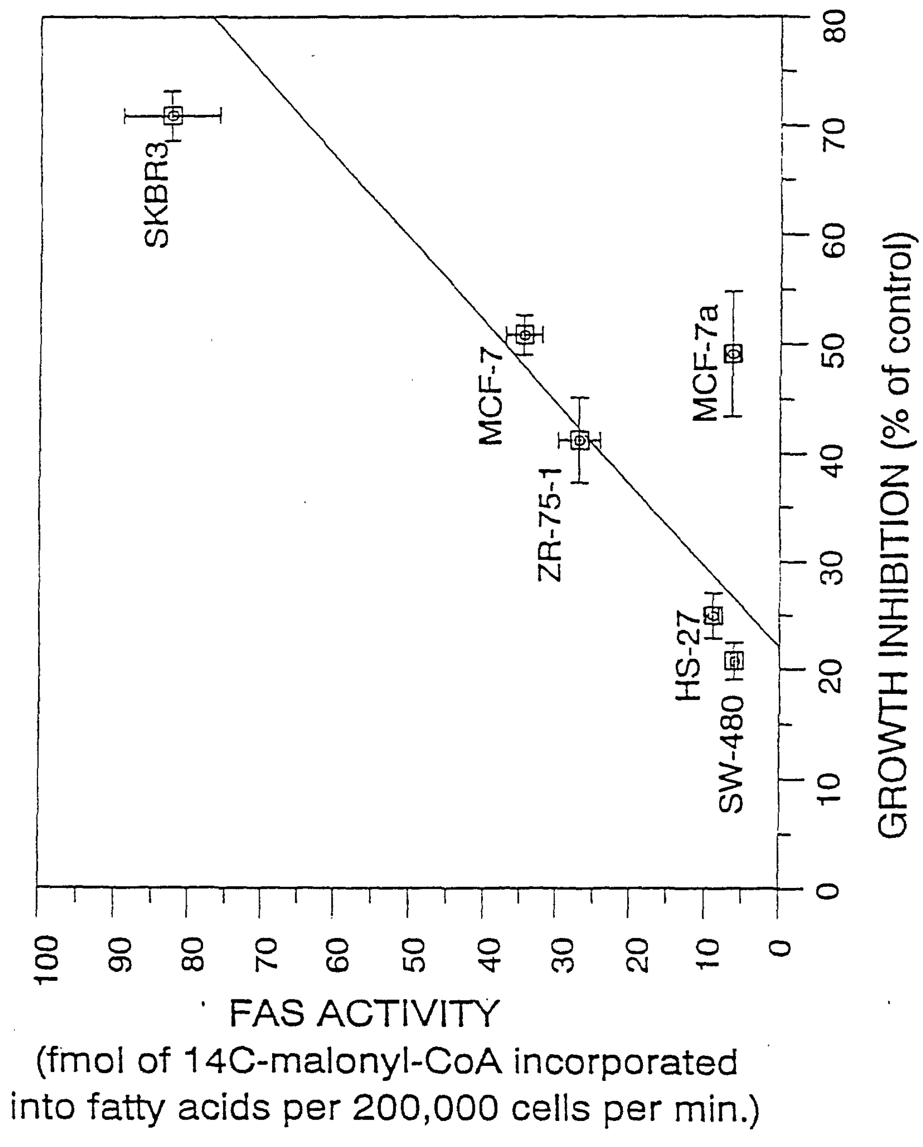

FIG. 12A

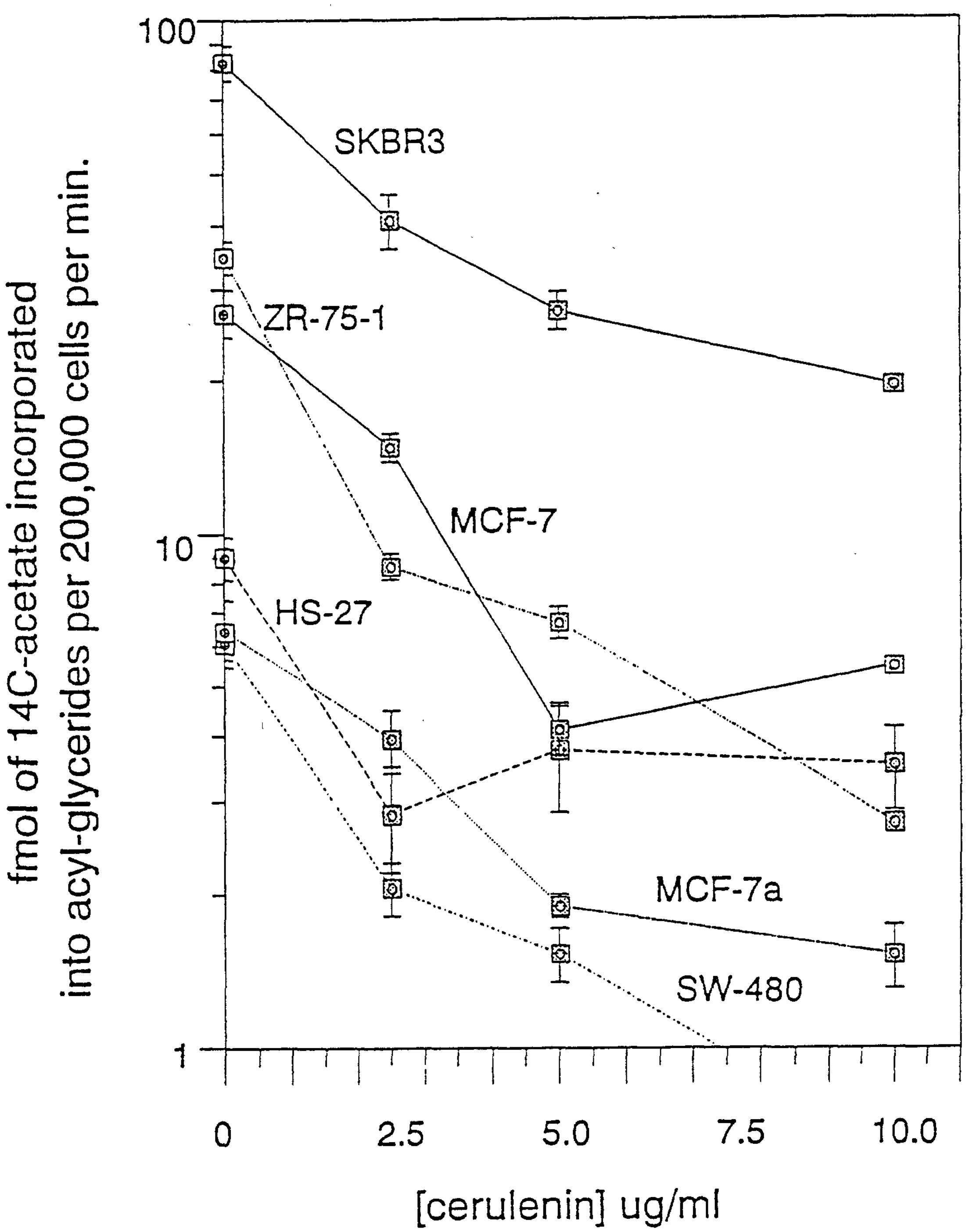
100
10
1
fmol of 14C-acetate incorporated into acyl-glycerides per 200,000 cells per min.
SKBR3
ZR-75-1
MCF-7
HS-27
MCF-7a
SW-480
0
2.5
5.0
7.5
10.0
[cerulenin] ug/ml

FIG. 12B

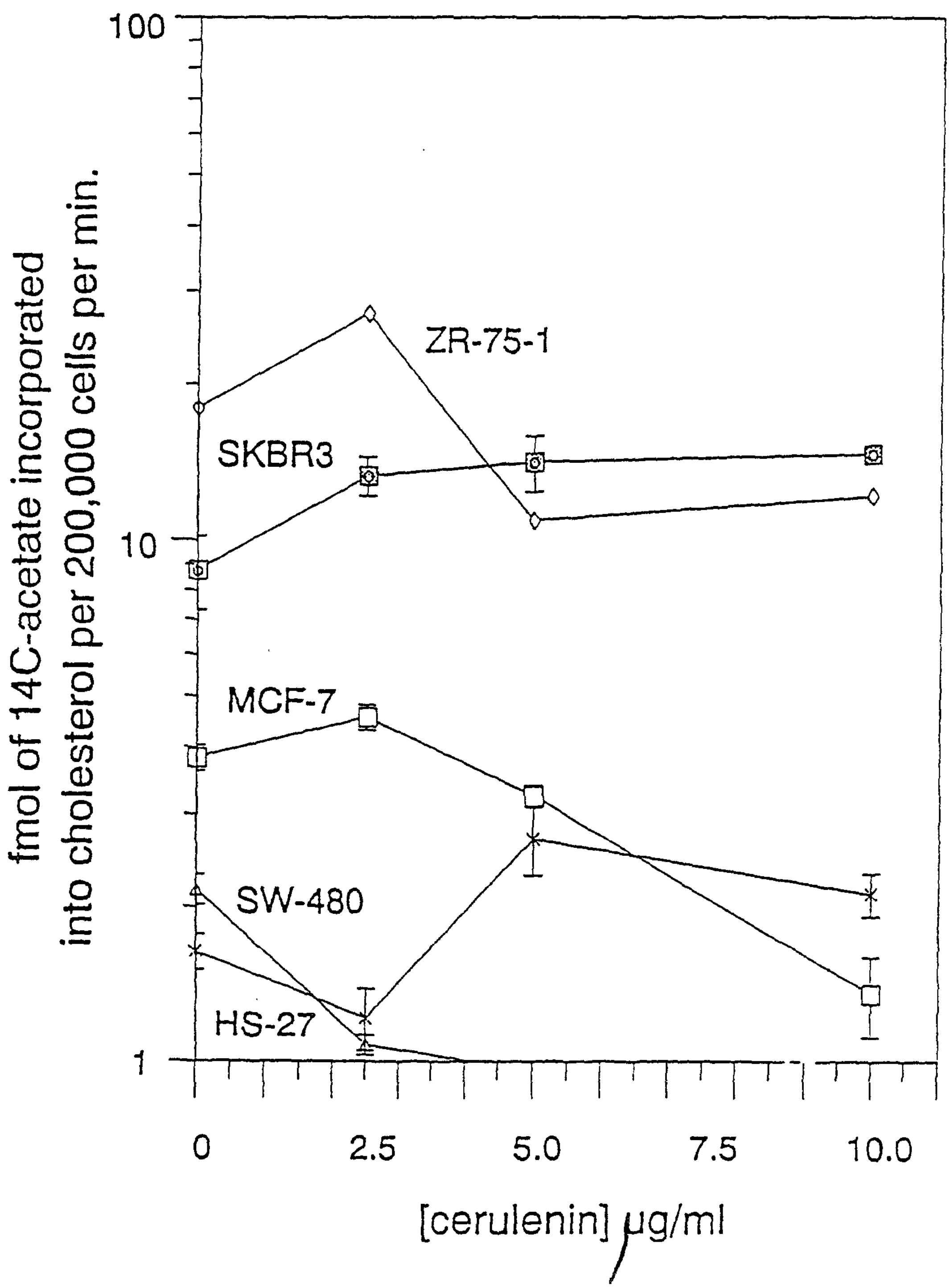
100
10
1
fmol of 14C-acetate incorporated into cholesterol per 200,000 cells per min.
ZR-75-1
SKBR3
MCF-7
SW-480
HS-27
0
2.5
5.0
7.5
10.0
[cerulenin] μg/ml

FIG. 13

Lack of Correlation Between Cell Doubling Times and Degree of Growth Inhibition by Cerulenin

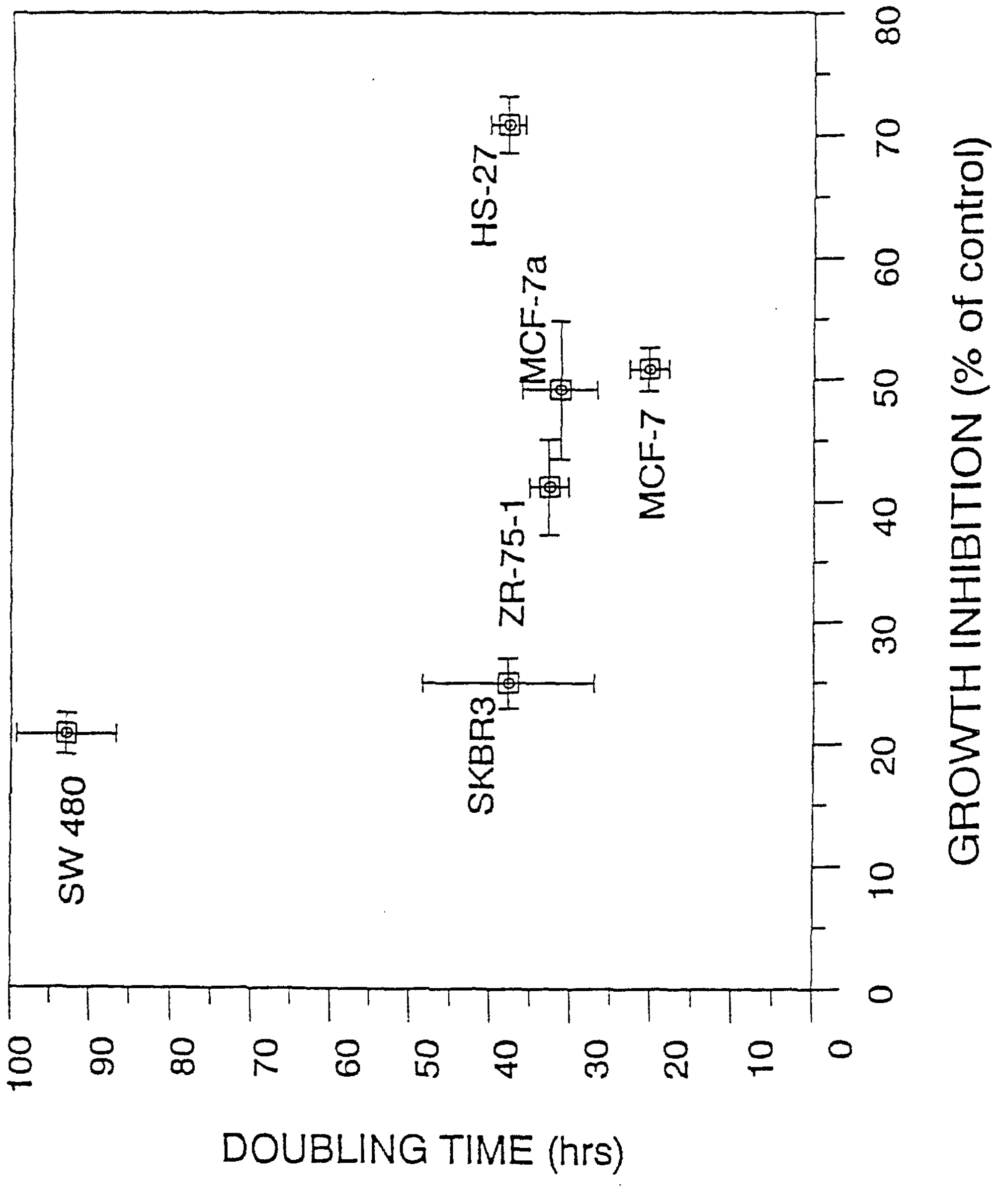

FIG. 14

POTENCY OF GROWTH INHIBITION OF HUMAN PROSTATE CANCER LINES BY CERULENIN

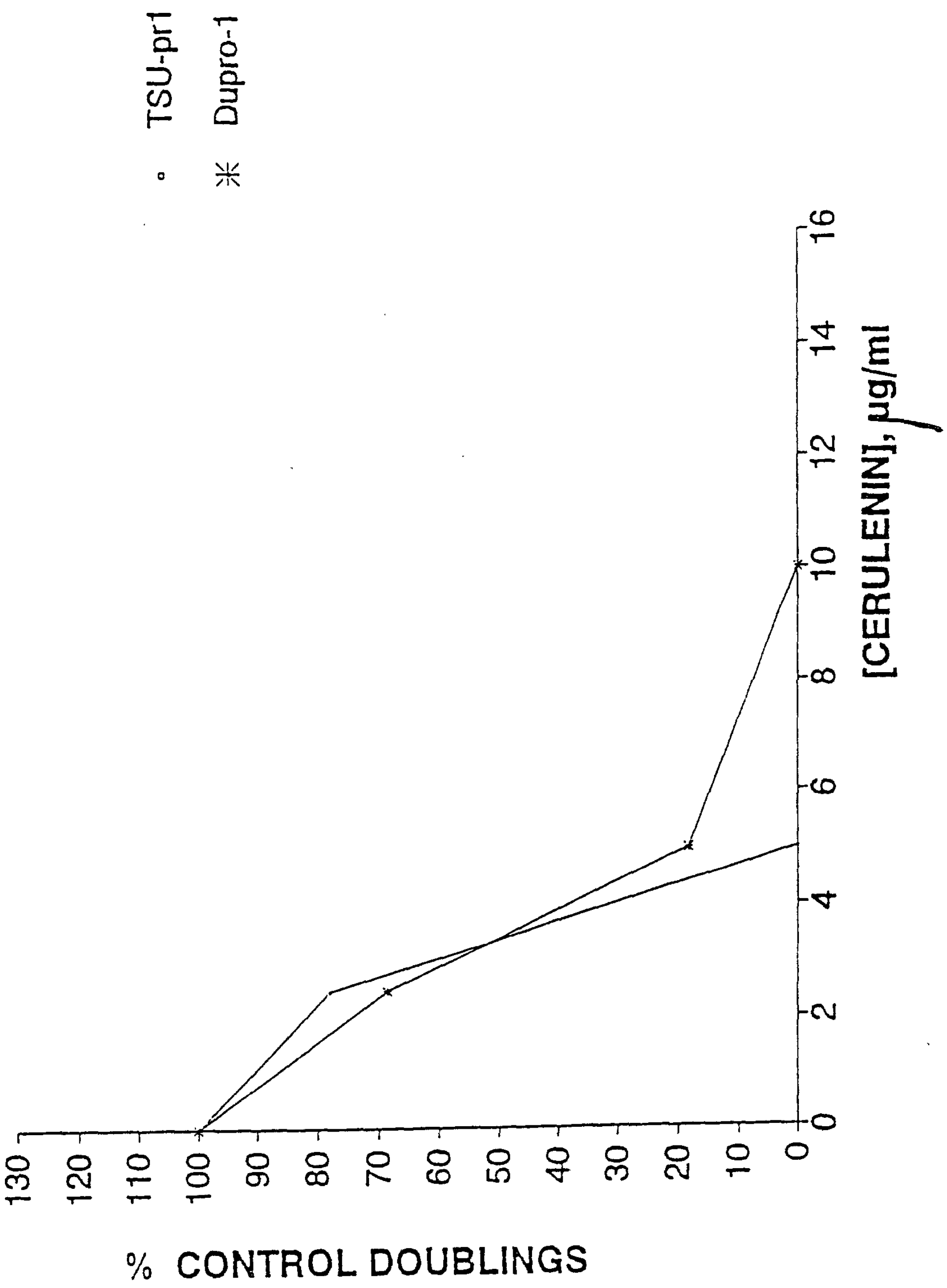

FIG. 15

POTENCY OF GROWTH INHIBITION BY TOFA

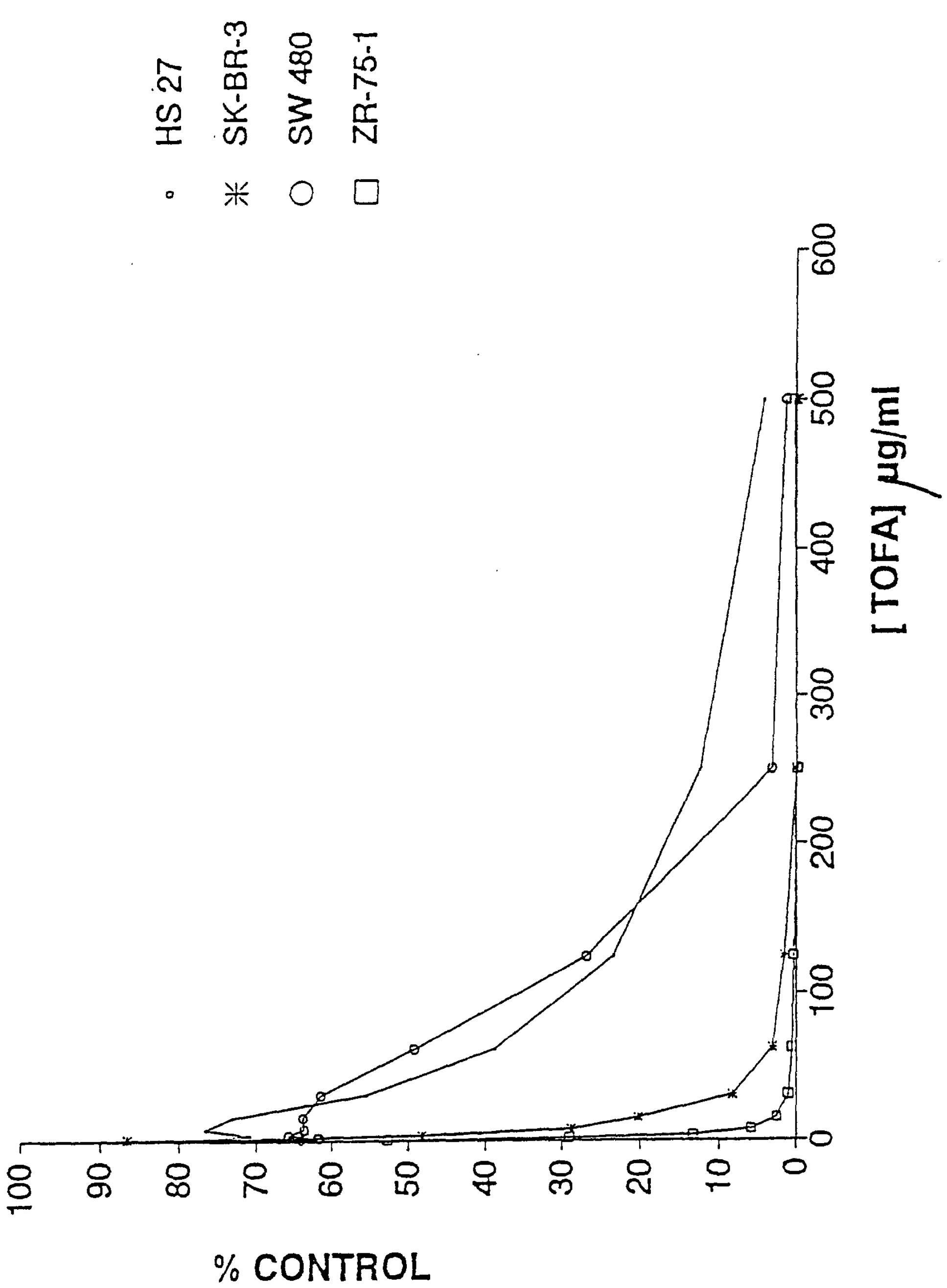

FIG. 16

Cerulenin Increases Degree of Growth Inhibition by Triacsin-C

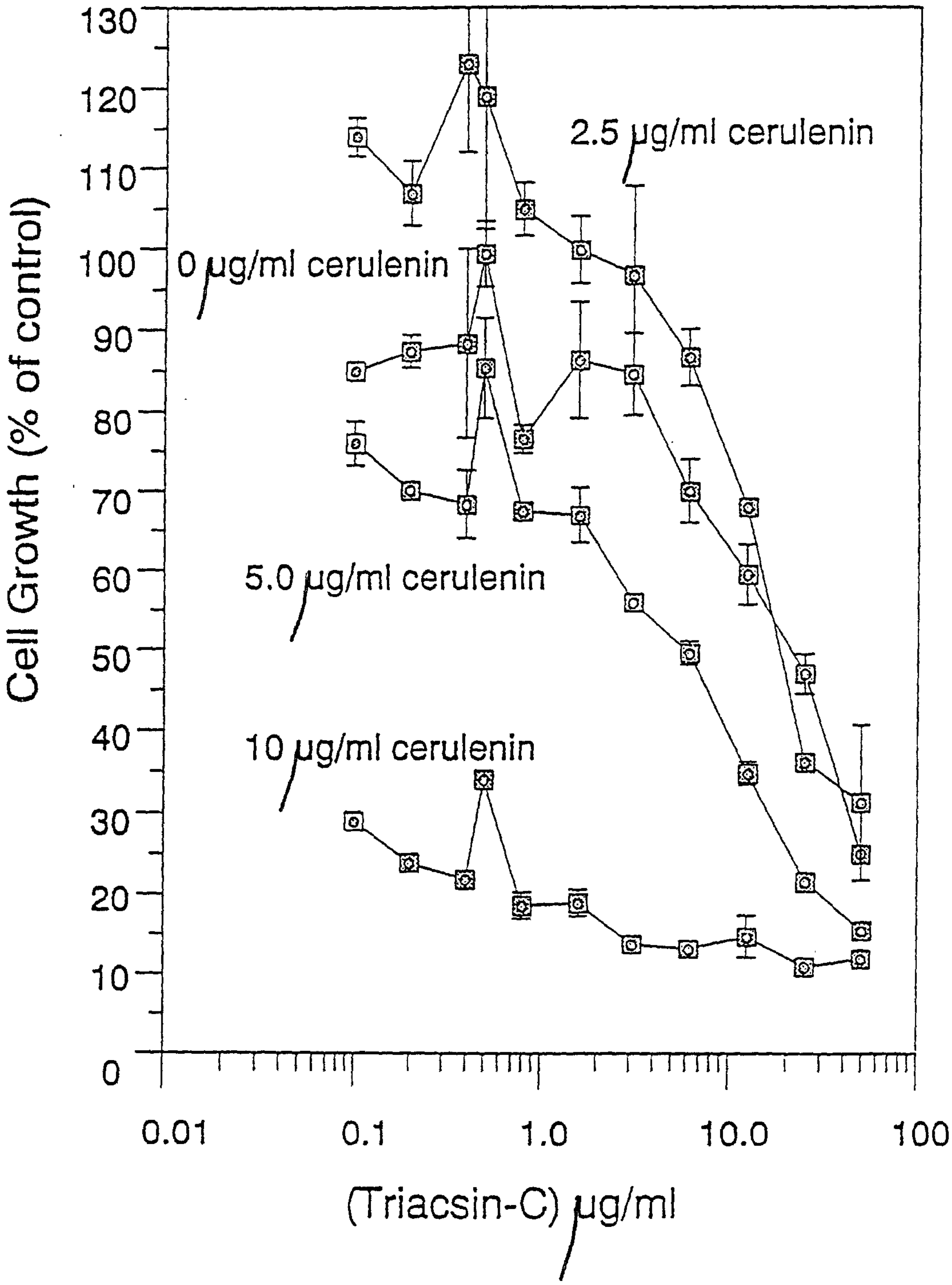

FIG. 17

RESCUE OF HUMAN MAMMARY ZR-75-1 CELLS FROM CERULENIN GROWTH INHIBITION BY EXOGENOUS OLEATE